# EUROPEAN PATENT APPLICATION

(11) **EP 2 036 923 A1**
(43) Date of publication of application: **18.03.2009**
(21) Application number: 07116067.5
(22) Date of filing: 11.09.2007
(51) Int. Cl.: C07K 14/575, A61K 38/00

(54) **Improved derivates of amylin**

(71) Applicant: NOVO NORDISK A/S, 2880 Bagsvaerd (DK)
(72) Inventor: The designation of the inventor has not yet been filed

(57) **Abstract**

The invention relates to derivatives of human amylin or analogues thereof which bind the amylin receptor and are linked to either a polyethylene glycol (PEG) polymer or to an albumin binding compound, pharmaceutical compositions comprising these derivatives and methods for obtaining such.

## Description

### FIELD OF THE INVENTION

The invention relates to derivatives of human amylin or analogues thereof which bind to albumin and/or the amylin receptor, pharmaceutical compositions comprising these derivatives, methods for obtaining such and amylin derivatives for use as medicaments.

### BACKGROUND OF THE INVENTION

A large and growing number of people suffer from diabetes mellitus and obesity. Diabetes mellitus is a metabolic disorder in which the ability to utilize glucose is partly or completely lost.

A number of treatment regimes are targeting excessive blood glucose whereas others are focused primarily on weight reduction. The most efficient anti-diabetic agent used to lower blood glucose is insulin and analogue(s) thereof. It has been known for a long time that when traditional insulin is used to treat diabetes, it is associated with an increase in body weight. Insulin has to be injected subcutaneously up to several times per day.

Type 2 diabetes is generally treated in the early phases with diet and exercise. As the condition progresses, various oral anti-diabetic agents are added. Injected agents such as GLP-1 analogues may also be used at this stage. In general, these agents are most efficient in patients with functioning beta-cells capable of releasing insulin and amylin.

Human amylin is a 37 amino acid long peptide which has physico-chemical properties that make its use as a drug troublesome. In particular, it has a tendency to fibrillate in-vitro and/or ex-vivo and become ineffective due to precipitation. A drug product called Symlin is currently on the market which contains an analogue of human amylin (pramlintide) where three of the 37 amino acids are substituted to proline. This improves substantially the fibrillating tendency. However, even pramlintide is difficult to keep in solution at neutral pH and it is therefore provided in an acidic solution i.e. Symlin.

Various amylin analogues and derivatives with improved PK/PD properties have been described. However; there is still a need to provide analogues and derivatives that have the activities of native human amylin, as well as derivatives which have a protracted PK-profile, show enhanced solubility and/or stability over native human amylin.

### SUMMARY OF THE INVENTION

The invention relates in one aspect to a derivative of human amylin or an analogue thereof, wherein
a) the amino acid residue in position 1 is any natural amino acid which is connected to a N-terminal extension consisting of 1-10 amino acids, wherein
   a. said extension is further linked to an albumin binding residue or a polyethylene glycol polymer, or
   b. an amino acid residue in position 2-37 has been substituted with a lysine residue, and said lysine residue is linked to an albumin binding residue or a polyethylene glycol polymer, optionally via a linker;
   or
b) an amino acid residue in position 3, 21, 25 or 29 has been substituted with a lysine residue and wherein said lysine residue is linked to an albumin binding residue or a polyethylene glycol polymer, optionally via a linker;
   or
c) the amino acid residue in position 18 is arginine, and an amino acid residue in position 1-17 or 19-37 has been substituted with a lysine residue , and wherein said lysine residue is linked to an albumin binding residue or a polyethylene glycol polymer, optionally via a linker;
wherein the amino acid numbering conforms with the amino acid numbering in SEQ ID NO:17.

The invention relates in a further aspect to a method for increasing the time of action in a patient of human amylin or an analog thereof, characterized by modifying
a) the amino acid residue in position 1 by optionally substituting with any natural amino acid, and by connecting said natural amino acid to a N-terminal extension consisting of 1-10 amino acids, and further
   a. linking said extension to an albumin binding residue or a polyethylene glycol polymer, or
   b. modifying an amino acid residue in position 2-37 by substitution with a lysine residue, and by linking said lysine residue to an albumin binding residue or a polyethylene glycol polymer, optionally via a linker,
   or;
b) the amino acid residue in position 3, 21, 25 or 29 by substitution with a lysine residue and by linking said lysine residue to an albumin binding residue or a polyethylene glycol polymer, optionally via a linker, or;
c) the amino acid residue in position 18 by substitution with an arginine residue and an amino acid residue in position 2-17 or 19-37 by substitution with a lysine residue and by linking said lysine residue to an albumin binding residue or a polyethylene glycol polymer, optionally via a linker;
wherein the amino acid numbering conforms with the amino acid numbering in SEQ ID NO:17.

The present invention also provides pharmaceutical compositions comprising a derivative according to the present invention and the use of the derivatives according to the present invention for preparing medicaments for treating diseases.

### DESCRIPTION OF THE INVENTION

It is the object of the invention to provide derivatives of human amylin or analogues thereof which bind to albumin and/or the amylin receptor.

In one aspect of the invention a derivative of human amylin or an analogue thereof is provided, wherein
a) the amino acid residue in position 1 is any natural amino acid which is connected to a N-terminal extension consisting of 1-10 amino acids, wherein
   a. said extension is further linked to an albumin binding residue or a polyethylene glycol polymer, or
   b. an amino acid residue in position 2-37 has been substituted with a lysine residue, and said lysine residue is linked to an albumin binding residue or a polyethylene glycol polymer, optionally via a linker;
   or
b) an amino acid residue in position 3, 21, 25 or 29 has been substituted with a lysine residue and wherein said lysine residue is linked to an albumin binding residue or a polyethylene glycol polymer, optionally via a linker;
   or
c) the amino acid residue in position 18 is arginine, and an amino acid residue in position 1-17 or 19-37 has been substituted with a lysine residue , and wherein said lysine residue is linked to an albumin binding residue or a polyethylene glycol polymer, optionally via a linker;
wherein the amino acid numbering conforms with the amino acid numbering in SEQ ID NO: 17.

In one aspect of the invention a derivative of human amylin or an analogue thereof is provided, wherein
a) the amino acid residue in position 1 is any natural amino acid which is connected to a N-terminal extension consisting of 1-10 amino acids, wherein
   a. said extension is further linked to an albumin binding residue or a polyethylene glycol polymer, or
   b. an amino acid residue in position 2-37 has been substituted with a lysine residue, and said lysine residue is linked to an albumin binding residue or a polyethylene glycol polymer, optionally via a linker;
   or
b) the amino acid residue in position 3 has been substituted with a lysine residue and wherein said lysine residue is linked to an albumin binding residue or a polyethylene glycol polymer, optionally via a linker;
   or
c) an amino acid residue in position 21, 25 or 29 has been substituted with a lysine residue and wherein said lysine residue is linked to an albumin binding residue or a polyethylene glycol polymer, optionally via a linker;
   or
d) the amino acid residue in position 18 is arginine, and an amino acid residue in position 1-17 or 19-37 has been substituted with a lysine residue , and wherein said lysine residue is linked to an albumin binding residue or a polyethylene glycol polymer, optionally via a linker;
wherein the amino acid numbering conforms with the amino acid numbering in SEQ ID NO: 17.

In one aspect of the invention a derivative of human amylin or an analogue thereof is provided, wherein
the amino acid residue in position 1 is any natural amino acid which is connected to a N-terminal extension consisting of 1-10 amino acids, wherein
a. said extension is further linked to an albumin binding residue or a polyethylene glycol polymer, or
b. an amino acid residue in position 2-37 has been substituted with a lysine residue, and said lysine residue is linked to an albumin binding residue or a polyethylene glycol polymer, optionally via a linker;
wherein the amino acid numbering conforms with the amino acid numbering in SEQ ID NO: 17.

In one aspect of the invention a derivative of human amylin or an analogue thereof is provided, wherein
the amino acid residue in position 1 is glutamic acid, and wherein said glutamic acid is connected to a N-terminal extension consisting of 1-10 amino acids, wherein said extension is further linked to an albumin binding residue or a polyethylene glycol polymer;
wherein the amino acid numbering conforms with the amino acid numbering in SEQ ID NO: 17.

In one aspect of the invention a derivative of human amylin or an analogue thereof is provided, wherein
the amino acid residue in position 1 is glutamic acid, and wherein said glutamic acid is connected to a N-terminal extension consisting of 1-6 amino acids, wherein said extension is further linked to an albumin binding residue or a polyethylene glycol polymer;
wherein the amino acid numbering conforms with the amino acid numbering in SEQ ID NO: 17.

In one aspect of the invention a derivative of human amylin or an analogue thereof is provided, wherein
the amino acid residue in position 1 is glutamic acid, and wherein said glutamic acid is connected to a N-terminal extension consisting of 5 amino acids, 4 amino acids, 3 amino acids, 2 amino acids or 1 amino acid, wherein said extension is further linked to an albumin binding residue or a polyethylene glycol polymer;
wherein the amino acid numbering conforms with the amino acid numbering in SEQ ID NO:17.

In one aspect of the invention a derivative of human amylin or an analogue thereof is provided, wherein
the amino acid residue in position 1 is any natural amino acid which is connected to a N-terminal extension consisting of 1-10 amino acids, and an amino acid residue in position 2-37 has been substituted with a lysine residue, wherein said lysine residue is linked to an albumin binding residue or a polyethylene glycol polymer, optionally via a linker;
wherein the amino acid numbering conforms with the amino acid numbering in SEQ ID NO:17.

In one aspect a derivative of human amylin or an analogue thereof is provided, wherein
the amino acid residue in position 3 has been substituted with a lysine residue and wherein said lysine residue is linked to an albumin binding residue or a polyethylene glycol polymer, optionally via a linker;
wherein the amino acid numbering conforms with the amino acid numbering in SEQ ID NO: 17.

In one aspect a derivative of human amylin or an analogue thereof is provided, wherein
an amino acid residue in position 21, 25 or 29 has been substituted with a lysine residue and wherein said lysine residue residue is linked to an albumin binding residue or a polyethylene glycol polymer, optionally via a linker;
wherein the amino acid numbering conforms with the amino acid numbering in SEQ ID NO:17.

In one aspect of the invention a derivative of human amylin or an analogue thereof is provided, wherein
the amino acid residue in position 21 has been substituted with a lysine residue and wherein said lysine residue is linked to an albumin binding residue or a polyethylene glycol polymer, optionally via a linker;
wherein the amino acid numbering conforms with the amino acid numbering in SEQ ID NO:17.

In one aspect of the invention a derivative of human amylin or an analogue thereof is provided, wherein
the amino acid residue in position 29 has been substituted with a lysine residue and wherein said lysine residue aspect is linked to an albumin binding residue or a polyethylene glycol polymer, optionally via a linker;
wherein the amino acid numbering conforms with the amino acid numbering in SEQ ID NO:17.

In one aspect a derivative of human amylin or an analogue thereof is provided, wherein
the amino acid residue in position 18 is arginine, and an amino acid residue in position 1-17 or 19-37 has been substituted with a lysine residue , and wherein said lysine residue is linked to an albumin binding residue or a polyethylene glycol polymer, optionally via a linker;
wherein the amino acid numbering conforms with the amino acid numbering in SEQ ID NO:17.

The following is a non-limiting list of aspects, which is further described elsewhere herein:
1. A derivative of human amylin with SEQ ID NO:17 or an analogue thereof, wherein
   a) the amino acid residue in position 1 is any natural amino acid which is connected to a N-terminal extension consisting of 1-10 amino acids, wherein
      a. said extension is further linked to an albumin binding residue or a polyethylene glycol polymer, or
      b. an amino acid residue in position 2-37 has been substituted with a lysine residue, and said lysine residue is linked to an albumin binding residue or a polyethylene glycol polymer, optionally via a linker;
      or
   b) the amino acid residue in position 3, 21, 25 or 29 has been substituted with a lysine residue and wherein said lysine residue is linked to an albumin binding residue or a polyethylene glycol polymer, optionally via a linker;
      or
   c) the amino acid residue in position 18 is arginine, and an amino acid residue in position 1-17 or 19-37 has been substituted with a lysine residue, and wherein said lysine residue is linked to an albumin binding residue or a polyethylene glycol polymer, optionally via a linker;
   wherein the amino acid numbering conforms with the amino acid numbering in SEQ ID NO:17.
2. A derivative of human amylin with SEQ ID NO: 17 or an analogue thereof according to aspect 1, wherein
   a) the amino acid residue in position 1 is any natural amino acid which is connected to a N-terminal extension consisting of 1-10 amino acids, wherein
      a. said extension is further linked to an albumin binding residue or a polyethylene glycol polymer, or
      b. an amino acid residue in position 2-37 has been substituted with a lysine residue, and said lysine residue is linked to an albumin binding residue or a polyethylene glycol polymer, optionally via a linker;
      or
   b) the amino acid residue in position 3 has been substituted with a lysine residue and wherein said lysine residue is linked to an albumin binding residue or a polyethylene glycol polymer, optionally via a linker;
      or
   c) an amino acid residue in position 21, 25 or 29 has been substituted with a lysine residue and wherein said lysine residue is linked to an albumin binding residue or a polyethylene glycol polymer, optionally via a linker;
      or
   d) the amino acid residue in position 18 is arginine, and an amino acid residue in position 1-17 or 19-37 has been substituted with a lysine residue, and wherein said lysine residue is linked to an albumin binding residue or a polyethylene glycol polymer, optionally via a linker;
   wherein the amino acid numbering conforms with the amino acid numbering in SEQ ID NO: 17.
3. A derivative of human amylin with SEQ ID NO:17 or an analogue thereof according to aspect 1 or 2, wherein
   the amino acid residue in position 1 is any natural amino acid which is connected to a N-terminal extension consisting of 1-10 amino acids, wherein
   a) said extension is further linked to an albumin binding residue or a polyethylene glycol polymer, or
   b) an amino acid residue in position 2-37 has been substituted with a lysine residue, and said lysine residue is linked to an albumin binding residue or a polyethylene glycol polymer, optionally via a linker;
   and optionally the amino acid residue in position 18 is arginine;
   wherein the amino acid numbering conforms with the amino acid numbering in SEQ ID NO:17.
4. A derivative of human amylin with SEQ ID NO: 17 or an analogue thereof according to aspect 1 or 2, wherein
   the amino acid residue in position 3, 21, 25 or 29 has been substituted with a lysine residue and wherein said lysine residue is linked to an albumin binding residue or a polyethylene glycol polymer, optionally via a linker;
   and optionally the amino acid residue in position 18 is arginine;
   wherein the amino acid numbering conforms with the amino acid numbering in SEQ ID NO:17.
5. The derivative according to aspect 1 or 2, wherein the derivative is human amylin of SEQ ID NO:17, wherein
   a) the amino acid residue in position 1 is any natural amino acid which is connected to a N-terminal extension consisting of 1-10 amino acids, wherein
      a. said extension is further linked to an albumin binding residue or a polyethylene glycol polymer, or
      b. an amino acid residue in position 2-37 has been substituted with a lysine residue, and said lysine residue is linked to an albumin binding residue or a polyethylene glycol polymer, optionally via a linker;
      or
   b) an amino acid residue in position 3, 21, 25 or 29 has been substituted with a lysine residue and wherein said lysine residue is linked to an albumin binding residue or a polyethylene glycol polymer, optionally via a linker;
      or
   c) the amino acid residue in position 18 is arginine, and an amino acid residue in position 1-17 or 19-37 has been substituted with a lysine residue, and wherein said lysine residue is linked to an albumin binding residue or a polyethylene glycol polymer, optionally via a linker.
6. The derivative according to any one of aspects 1-4, wherein the derivative is human amylin of SEQ ID No 17 or an analogue thereof, wherein
   an amino acid residue in position 3 has been substituted with a lysine residue and wherein such a lysine residue is linked to an albumin binding residue or a polyethylene glycol polymer, optionally via a linker.
7. The derivative according to aspect 6, wherein the derivative is human amylin of SEQ ID NO: 17 or an analogue thereof, wherein
   an amino acid residue in position 3 has been substituted with a lysine residue and wherein such a lysine residue is linked to an albumin binding residue, optionally via a linker
8. The derivative according to any one of aspects 1-4, wherein the derivative is human amylin of SEQ ID NO:17 or an analogue thereof, wherein
   an amino acid residue in position 21 has been substituted with a lysine residue and wherein such a lysine residue is linked to an albumin binding residue or a polyethylene glycol polymer, optionally via a linker.
9. The derivative according to any one of aspects 1-4, wherein the derivative is human amylin of SEQ ID NO:17 or an analogue thereof, wherein
   an amino acid residue in position 25 has been substituted with a lysine residue and wherein such a lysine residue is linked to an albumin binding residue or a polyethylene glycol polymer, optionally via a linker.
10. The derivative according to any one of aspects 1-4, wherein the derivative is human amylin of SEQ ID NO: 17 or an analogue thereof, wherein
   an amino acid residue in position 29 has been substituted with a lysine residue and wherein such a lysine residue is linked to an albumin binding residue or a polyethylene glycol polymer, optionally via a linker.
11. The derivative according to any one of aspects 1-4, wherein the derivative is human amylin of SEQ ID NO:17 or an analogue thereof, wherein
   the amino acid in position 18 is arginine, and an amino acid residue in position 1-17 or 19-37 has been substituted with a lysine residue , and wherein such a lysine residue is linked to an albumin binding residue or a polyethylene glycol polymer, optionally via a linker.
12. The derivative according to any one of aspects 1-11, wherein the derivative has from 1-12 amino acid substitutions compared to human amylin.
13. The derivative according to any one of aspects 1-12, wherein the derivative has from 1-10 amino acid substitutions compared to human amylin.
14. The derivative according to any one of aspects 1-13, wherein the derivative has from 1-8 amino acid substitutions compared to human amylin.
15. The derivative according to any one of aspects 1-14, wherein the derivative has from 1-6 amino acid substitutions compared to human amylin.
16. The derivative according to any one of aspects 1-15, wherein the derivative has from 1-4 amino acid substitutions compared to human amylin.
17. The derivative according to any one of aspects 1-16, wherein the derivative is an analogue of human amylin of SEQ ID NO:17, wherein 0-8 additional charges have been added compared to human amylin.
18. The derivative according to aspect 17, wherein the 0-8 additional charges have been added by substituting one or more amino acid residues of human amylin or analogue thereof with charged amino acids and/or by adding charged amino acids in a N-terminal extention, or by adding negatively charged entities in the albumin binding residue or polyethylene glycol polymer and/or the linker.
19. The derivative according to aspect 17 or 18, wherein the 0-8 additional charges have been added by substituting one or more amino acid residues of human amylin or an analogue thereof with glutamic acid residue(s) and/or aspartic acid residue(s) and/or by adding charged amino acids in a N-terminal extention.
20. The derivative according any of the aspects 17-19, wherein the 0-8 additional charges have been added by adding charged amino acids in a N-terminal extension.
21. The derivative according to any of the aspects 1-3, 5 or 12-20, wherein the derivative comprises an N-terminal extension consisting of 1-10 amino acids, wherein said extension is further linked to an albumin binding residue or a polyethylene glycol polymer.
22. The derivative according to aspect 21, wherein the derivative comprises an N-terminal extension consisting of 1-8 amino acids, wherein said extension is further linked to an albumin binding residue or a polyethylene glycol polymer.
23. The derivative according to aspect 22, wherein the derivative comprises an N-terminal extension consisting of 1-6 amino acids, wherein said extension is further linked to an albumin binding residue or a polyethylene glycol polymer.
24. The derivative according to aspect 23, wherein the derivative comprises an N-terminal extension consisting of 5 amino acids, wherein said extension is further linked to an albumin binding residue or a polyethylene glycol polymer.
25. The derivative according to aspect 24, wherein the derivative comprises an N-terminal extension consisting of 4 amino acids, wherein said extension is further linked to an albumin binding residue or a polyethylene glycol polymer.
26. The derivative according to aspect 25, wherein the derivative comprises an N-terminal extension consisting of 3 amino acids, wherein said extension is further linked to an albumin binding residue or a polyethylene glycol polymer.
27. The derivative according to aspect 26, wherein the derivative comprises an N-terminal extension consisting of 2 amino acids, wherein said extension is further linked to an albumin binding residue or a polyethylene glycol polymer.
28. The derivative according to aspect 27, wherein the derivative comprises an N-terminal extension consisting of 1 amino acid, wherein said extension is further linked to an albumin binding residue or a polyethylene glycol polymer.
29. A derivative according to aspect 1 comprising an amino acid sequence of formula 1: 
Xaa1-Cys- Xaa3-Thr-Ala-Thr-Cys-Ala-Thr-Gln-Arg-Leu-Ala-Asn-Phe-Leu- Xaa17-Xaa18-
Ser-Ser- Xaa21-Asn- Xaa23-Gly- Xaa25- Xaa26-Leu-Xaa28-Xaa29-Thr-Asn-Val-Gly-Ser-Asn-
Thr-Tyr Formula (1) (SEQ ID No:18)
 wherein
   Xaa₁ is deleted or independently selected from Lys and Glu;
   Xaa₃ is independently selected from Asn, Gly and Lys;
   Xaa₁₇ is independently selected from Ala, Val and Lys;
   Xaa₁₈ is independently selected from His, Pro and Arg;
   Xaa₂₁ is independently selected from Asn, Gln and Lys;
   Xaa₂₃ is independently selected from Phe and Leu;
   Xaa₂₅ is independently selected from Ala, Pro and Lys;
   Xaa₂₆ is independently selected from Val and Ile;
   Xaa₂₈ is indenpendently selected from Ser and Pro;
   Xaa₂₉ is independently selected from Ser, Pro and Lys;
   the C-terminal may optionally be derivatized as an amide;
   the N-terminal may optionally be extended with 1-10 amino acid residues
   wherein if Xaa₁ is Lys and Xaa₃ is Asn and Xaa₂₁ is Asn and Xaa₂₅ is Ala or Pro, then Xaa₂₉ is Lys; and
   if Xaa₁ is Glu, then said Glu is connected to a N-terminal extension consisting of 1-10 amino acids, and said extension is further linked to an albumin binding residue or a polyethylene glycol polymer, and
   if Xaa₁ is Lys, then one amino acid in a position selected from the group consisting of Xaa₁, Xaa₃, Xaa₂₁, Xaa₂₅ and Xaa₂₉ in formula (1) is linked to an albumin binding residue or a polyethylene glycol polymer, optionally via a linker.
30. The derivative according to any one of the aspects 1-29, wherein the derivative is linked to a polyethylene glycol polymer.
31. The derivative according to aspect 30, wherein the poly ethylene glycol polymer is a polyethylene glycol having a molecular weight of at least 30 kD.
32. The derivative according to any one of the aspects 30-31, wherein the polyethylene glycol polymer is a polyethylene glycol which is branched.
33. The derivative according to any one of the aspects 1-29, wherein the derivative is linked to an albumin binding residue.
34. The derivative according to aspect 33, wherein the albumin binding residue is a lipophilic residue.
35. The derivative according to aspect 33, wherein the albumin binding residue is negatively charged at physiological pH.
36. The derivative according to aspect 33, wherein the albumin binding residue comprises a group which can be negatively charged.
37. The derivative according to aspect 36, wherein the albumin binding residue comprises a carboxylic acid group.
38. The derivative according to any one of the aspects 33-37, wherein the albumin binding residue binds non-covalently to albumin.
39. The derivative according to any one of the aspects 33-38, wherein the albumin binding residue has a binding affinity towards human serum albumin that is below about 10 µM or below about 1 µM.
40. The derivative according to aspect 33, wherein the albumin binding residue is selected from the group consisting of a straight chain alkyl group, a branched alkyl group, a group which has an ω-carboxylic acid group, and a partially or completely hydrogenated cyclopentanophenanthrene skeleton.
41. The derivative according to aspect 33, wherein the albumin binding residue is a cibacronyl residue.
42. The derivative according to aspect 34, wherein the lipophilic residue comprises a partially or completely hydrogenated cyclopentanophenathrene skeleton.
43. The derivative according to aspect 34, wherein the albumin binding residue has from 6 to 40 carbon atoms, from 8 to 26 carbon atoms or from 8 to 20 carbon atoms.
44. The derivative according to aspect 34, wherein the albumin binding residue is an acyl group selected from the group comprising CH₃(CH₂)ᵣCO-, wherein r is an integer from 4 to 38, preferably an integer from 4 to 24, more preferred selected from the group comprising CH₃(CH₂)₆CO-, CH₃(CH₂)₈CO-, CH₃(CH₂)₁₀CO-, CH₃(CH₂)₁₂CO-, CH₃(CH₂)₁₄CO-, CH₃(CH₂)₁₆CO-, CH₃(CH₂)₁₈CO-, CH₃(CH₂)₂₀CO- and CH₃(CH₂)₂₂CO-.
45. The derivative according to aspect 34, wherein the albumin binding residue is an acyl group of a straight-chain or branched alkane α,ω-ordicarboxylic acid.
46. The derivative according to aspect 34, wherein the albumin binding residue is an acyl group selected from the group comprising HOOC(CH₂)ₛCO-, wherein s is an integer from 4 to 38, preferably an integer from 4 to 24, more preferred selected from the group comprising HOOC(CH₂)₁₄CO-, HOOC(CH₂)₁₆CO-, HOOC(CH₂)₁₈CO-, HOOC(CH₂)₂₀CO- and HOOC(CH₂)₂₂CO-.
47. The derivative according to aspect 34, wherein the albumin binding residue is a group of the formula CH₃(CH₂)ᵥCO-NHCH(COOH)(CH₂)₂CO-, wherein v is an integer from 10 to 24.
48. The derivative according to aspect 34, wherein the albumin binding residue is a group of the formula CH₃(CH₂)_{w}CO-NHCH((CH₂)₂COOH)CO-, wherein w is an integer from 8 to 24.
49. The derivative according to aspect 34, wherein the albumin binding residue is a group of the formula COOH(CH₂)xCO-, wherein x is an integer from 8 to 24.
50. The derivative according to aspect 34, wherein the albumin binding residue is a group of the formula -NHCH(COOH)(CH₂)₄NH-CO(CH₂)_{y}CH₃, wherein y is an integer from 8 to 18.
51. The derivative according to aspect 33, wherein the albumin binding residue is a peptide, such as a peptide comprising less than 40 amino acid residues.
52. The derivative according to any one of the aspects 33-51, wherein the albumin binding residue optionally via a linker is connected via the ε-amino group of a lysine residue.
53. A pharmaceutical composition comprising a derivative according to any one of the aspects 1-52, and a pharmaceutically acceptable excipient.
54. The pharmaceutical composition according to aspect 53, which is suited for parenteral administration.
55. A derivative according to any one of the aspects 1-52 for use as a medicament.
56. A derivative according to any one of the aspects 1-52 for use as a medicament for the treatment or prevention of hyperglycemia, type 2 diabetes, impaired glucose tolerance, type 1 diabetes, obesity, hypertension, syndrome X, dyslipidemia, cognitive disorders, atherosclerosis, myocardial infarction, coronary heart disease and other cardiovascular disorders, stroke, inflammatory bowel syndrome, dyspepsia and gastric ulcers.
57. A derivative according to any one of the aspects 1-52 for use as a medicament for delaying or preventing disease progression in type 2 diabetes.
58. A derivative according to any one of the aspects 1-52 for use as a medicament for decreasing food intake, decreasing β-cell apoptosis, increasing β-cell function and β-cell mass, and/or for restoring glucose sensitivity to β-cells. Use of a derivative according to any one of the aspects 1-42 for the preparation of a medicament.
59. Use of a derivative according to any one of the aspects 1-52 for the preparation of a medicament for the treatment or prevention of hyperglycemia, type 2 diabetes, impaired glucose tolerance, type 1 diabetes, obesity, hypertension, syndrome X, dyslipidemia, cognitive disorders, atherosclerosis, myocardial infarction, coronary heart disease and other cardiovascular disorders, stroke, inflammatory bowel syndrome, dyspepsia and gastric ulcers.
60. Use of a derivative according to any one of the aspects 1-52 for the preparation of a medicament for delaying or preventing disease progression in type 2 diabetes.
61. Use of a derivative according to any one of the aspects 1-52 for the preparation of a medicament for decreasing food intake, decreasing β-cell apoptosis, increasing β-cell function and β-cell mass, and/or for restoring glucose sensitivity to β-cells.
62. Method for increasing the time of action in a patient of human amylin or an analog thereof, characterized by modifying
   a) the amino acid residue in position 1 by optionally substituting with any natural amino acid, and by connecting said natural amino acid to a N-terminal extension consisting of 1-10 amino acids, and further
      a. linking said extension to an albumin binding residue or a polyethylene glycol polymer, or
      b. modifying an amino acid residue in position 2-37 by substitution with a lysine residue, and by linking said lysine residue to an albumin binding residue or a polyethylene glycol polymer, optionally via a linker,
      or;
   b) the amino acid residue in position 3, 21, 25 or 29 by substitution with a lysine residue and by linking said lysine residue to an albumin binding residue or a polyethylene glycol polymer, optionally via a linker,
      or;
   c) the amino acid residue in position 18 by substitution with an arginine residue and an amino acid residue in position 2-17 or 19-37 by substitution with a lysine residue and by linking said lysine residue to an albumin binding residue or a polyethylene glycol polymer, optionally via a linker;
   wherein the amino acid numbering conforms with the amino acid numbering in SEQ ID NO:17.
63. Method according to aspect 62, wherein the time of action in a patient of human amylin or an analog thereof is increased to more than about 40 hours.
64. Method according to any one of the aspects 62 or 63, wherein the human amylin or an analog thereof is as further defined in any one of the aspects 1-52.
65. A method for the treatment, prevention or alleviation of hyperglycemia, type 2 diabetes, impaired glucose tolerance, type 1 diabetes, obesity, syndrome X or dyslipidemia in a subject comprising administering to a subject a derivative according to any one of aspects 1-52 or a pharmaceutical composition according to aspects 53 or 54.
66. A process for preparing a pharmaceutical composition according to aspect 53 or 54 comprising mixing a derivative according to any one of aspects 1-52 with pharmaceutically acceptable substances and/or excipients.

In one aspect a derivative of human amylin or an analogue thereof is provided which shows high potency. In a further aspect a derivative of human amylin or an analogue thereof is provided which shows improved potency relative to human amylin. In a still further aspect a derivative of human amylin or an analogue thereof is provided which shows potency comparable to pramlintide. In a still further aspect a derivative of human amylin or an analogue thereof is provided which shows improved potency relative to pramlintide.

The term "potency" (or activity) is used to indicate the affinity to an amylin receptor of a derivative according to the invention and may be measured in e.g. a Luciferase assay as described in the assay section.

In one aspect a derivative of human amylin or an analogue thereof is provided which is physical stabile. In a further aspect a derivative of human amylin or an analogue thereof is provided which has increased physical stability relative to human amylin.

The term "physical stability" of a derivative according to the invention refers as used herein to the tendency of the protein to form biologically inactive and/or insoluble aggregates of the protein as a result of exposure of the protein to thermo-mechanical stresses and/or interaction with interfaces and surfaces that are destabilizing, such as hydrophobic surfaces and interfaces. Physical stability of the aqueous protein formulations may be evaluated by means of visual inspection, ThT fibrillation assay and/or turbidity measurements as described elsewhere herein.

The term "human amylin" as used herein refers to the peptide human amylin having the sequence as depicted in SEQ ID No 17. The term includes, but is not limited to, a human peptide hormone of 37 amino acids referred to as amylin, which is co-secreted with insulin from β-cells of the pancreas. Human amylin has the following amino acid sequence: Lys-Cys-Asn-Thr-Ala-Thr-Cys-Ala-Thr-Gln-Arg-Leu-Ala-Asn-Phe-Leu-Val-His-Ser-Ser-Asn-Asn-Phe-Gly-Ala-Ile-Leu-Ser-Ser-Thr-Asn-Val-Gly-Ser-Asn-Thr-Tyr (SEQ ID NO:17). Thus, the structural formula is Lys-Cys-Asn-Thr-Ala-Thr-Cys-Ala-Thr-Gln-Arg-Leu-Ala-Asn-Phe-Leu-Val-His-Ser-Ser-Asn-Asn-Phe-Gly-Ala-Ile-Leu-Ser-Ser-Thr-Asn-Val-Gly-Ser-Asn-Thr-Tyr-NH2 (SEQ ID NO: 17) as shown below with a disulfide bridge between the two Cys residues and a C-terminal amide group.

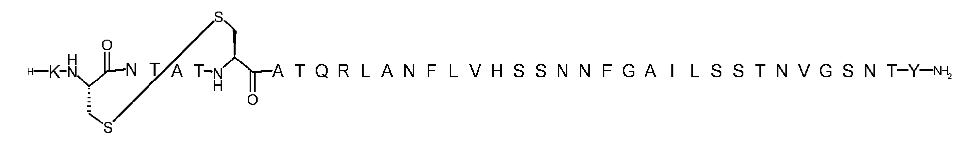

In the present text, the term "analogue of human amylin" is used to designate a peptide wherein one or more amino acid residues of human amylin of SEQ ID NO: 17 independently have been substituted by other amino acid residues and/or wherein one or more amino acid residues of human amylin have been deleted and/or wherein one or more amino acid residues have been added to human amylin. In one aspect a substitution or addition is with any natural amino acid.

When used herein the term "natural amino acid" is an amino acid (with the usual three letter codes & one letter codes in parenthesis) selected from the group consisting of: Glycine (Gly & G), proline (Pro & P), alanine (Ala & A), valine (Val & V), leucine (Leu & L), isoleucine (Ile & I), methionine (Met & M), cysteine (Cys & C), phenylalanine (Phe & F), tyrosine (Tyr & Y), tryptophan (Trp & W), histidine (His & H), lysine (Lys & K), arginine (Arg & R), glutamine (Gln & Q), asparagine (Asn & N), glutamic acid (Glu & E), aspartic acid (Asp & D), serine (Ser & S) and threonine (Thr & T). If, due to typing errors, there are deviations from the commonly used codes, the commonly used codes apply. The amino acids present in the insulins of this invention are, preferably, amino acids which can be coded for by a nucleic acid.

In one aspect of the invention, addition or deletion of amino acid residues can take place at the N-terminal of the peptide and/or at the C-terminal of the peptide.

In one aspect substitution or addition of amino acid residues comprises substitution and/or addition of glutamic acid residue(s), lysine residue(s), arginine residue(s), histidine residue(s) and/or aspartic acid residue(s) to obtain an amylin analogue having 0-8 additional charges compared to the parent amylin. In a further aspect substitution or addition of amino acid residues comprises substitution and/or addition of glutamic acid residue(s), arginine residue(s), and/or aspartic acid residue(s) to obtain an amylin analogue having 0-8 additional charges compared to the parent amylin. In a yet further aspect substitution or addition of amino acid residues comprises substitution and/or addition of glutamic acid residue(s) and/or arginine residue(s) to obtain an amylin analogue having 0-8 additional charges compared to the parent amylin. In a still further aspect substitution or addition of amino acid residues comprises substitution and/or addition of glutamic acid residue(s) to obtain an amylin analogue having 0-8 additional charges compared to the parent amylin.

An analogue of human amylin has in one aspect preferably from 30 to 45 naturally occurring or non-naturally occurring amino acids, preferably from 35-40 naturally occurring or non-naturally occurring amino acids.

The term "derivative" is used in the present text to designate a peptide in which one or more of the amino acid residues of the peptide have been modified, e.g. by alkylation, acylation, ester formation, amide formation or by maleimide coupling.

The term "a derivative of human amylin or an analogue thereof" or "an amylin derivative" is used in the present text to designate a derivative of human amylin or an analogue thereof.

In a further aspect of the invention, an analogue of human amylin has sufficient homology with human amylin such that it exhibits at least 70% *in vivo* activity compared to human amylin.

The derivatives of the present invention may be capable of binding to or otherwise directly or indirectly interacting with an amylin receptor, or other receptor or receptors with which amylin itself may interact to elicit a biological response, e.g., reducing food intake. Derivatives of the invention may bind an amylin receptor with an affinity better than 20nM, 10 nM, or 5 nM, and more preferably with an affinity better than 2 nM, 1 nM, 0.5 nM, 0.2 nM or 0.10 nM.

In one aspect according to the invention, the derivatives of the present invention may retain at least about 25%, preferably about 30%, 40%, 50%, 60%, 70%, 80%, 90%, 95%, 98%, or 99% percent of the biological activity of amylin. In another aspect, derivatives of the invention exhibit improved biological activity. In a further aspect, derivatives of the present invention exhibit at least about 110%, 125%, 130%, 140%, 150%, 200%, or more of the biological activity of amylin.

The term "activity" refers in one aspect to the ability to reduce appetite and/or increase satiety. In one aspect of the invention, activity is measured by the ability to reduce appetite as e.g. described in Pharmacological assays I and II under the heading "ASSAYS".

In one aspect according to the invention, the derivatives of the present invention may demonstrate an ability to reduce cumulative food intake more than 5% over administration of the vehicle, preferably more than 15%, more preferably more than 25%, even more preferably more than 35% or 40% most preferably more than 50% over the vehicle.

The term "activity" refers in another aspect to the ability to delay gastric emptying in an animal model. The term "activity" refers in another aspect to the ability to inhibit release of glucagon in an animal model.

The term "time of action" refers in the present context to the time span where a pharmacological effect such as reduced food intake is measurable.

In one aspect according to the invention, the amylin derivative is less likely to fibrillate in vivo and/or ex-vivo compared to human amylin. The tendency of fibrillation may e.g. be estimated in a Thioflavin T test as described below under the definition of the term "physical stability".

In one aspect an amylin derivative according to the invention has an incresed tendency to form an alfa helical containing conformation compared to pramlintide under similar conditions. The content of alfa helix is estimated by the magnitude of the negative molar circular dichroism signal at 220 nm, and further assinged by the classical CD spectral signatures for an alfa helix: A positive peak around 190 nm and a negative peak around 208 nm.

In one aspect according to the invention, the amylin derivative has a protracted pharmaco-kinetic profile compared to pramlintide as measured by standard procedures such as ELISA known to people skilled in the art. In another aspect of the invention, the amylin derivative has a plasma T½ of at least 1 hour. In another aspect, of the invention the plasma T½ is at least 1.5 hour. In another aspect of the invention, the amylin derivative has a plasma T½ of at least 2 hours. In another aspect of the invention, the amylin derivative has a plasma T½ of at least 4 hours. In another aspect of the invention, the amylin derivative has a plasma T½ of at least 6 hours. In another aspect of the invention, the amylin derivative has a plasma T½ of at least 8 hours. In another aspect of the invention, the amylin derivative has a plasma T½ of at least 12 hours. In another aspect of the invention, the amylin derivative has a plasma T½ of at least 16 hours. In another aspect of the invention, the amylin derivative has a plasma T½ of at least 24 hours. In one aspect of the invention, the amylin derivative has a plasma T½ of at least 48 hours. In one aspect of the invention, the amylin derivative has a plasma T½ of at least 96 hours.

In another further aspect of the invention, an analogue of human amylin has the amino acid sequence of human amylin modified so that from one, two, three, four, five, six, seven or eight amino acids differ from the amino acid in the corresponding position of human amylin. In another further aspect of the invention, an analogue comprises less than 8 modifications (substitutions, deletions, additions) relative to human amylin. In another further aspect of the invention, an analogue comprises less than 7 modifications (substitutions, deletions, additions) relative to human amylin. In another further aspect of the invention, an analogue comprises less than 6 modifications (substitutions, deletions, additions) relative to human amylin. In another further aspect of the invention, an analogue comprises less than 5 modifications (substitutions, deletions, additions) relative to human amylin. In another further aspect of the invention, an analogue comprises less than 4 modifications (substitutions, deletions, additions) relative to human amylin. In another further aspect of the invention, an analogue comprises less than 3 modifications (substitutions, deletions, additions) relative to human amylin. In another further aspect of the invention, an analogue comprises less than 2 modifications (substitutions, deletions, additions) relative to human amylin. In another further aspect of the invention, an analogue comprises only a single modification (substitutions, deletions, additions) relative to human amylin.

In one aspect of the invention, the modification(s) are substitution(s). In one aspect of the invention, the modification(s) are deletion(s). In one aspect of the invention, the modification(s) are addition(s).

In one aspect a derivative of human amylin or an analogue thereof is extended in the N-terminal. In a further aspect a derivative of human amylin or an analogue thereof is extended in the N-terminal, where the N-terminal extension consists of 1-10 amino acids, 1-8 amino acids or 1-6 amino acids. In a further aspect a derivative of human amylin or an analogue thereof is extended in the N-terminal, where the N-terminal extension consists of 5 amino acids, 4 amino acids, 3 amino acids, 2 amino acids or 1 amino acid. In a further aspect the N-terminal extension is further linked to an albumin binding residue or a polyethylene glycol polymer. In one aspect the N-terminal extension is further linked to an albumin binding residue or a polyethylene glycol polymer, where the N-terminal amino acid of said N-terminal extension is linked to said albumin binding residue or said polyethylene glycol polymer.

In one aspect the invention relates to a derivative of human amylin with SEQ ID No. 17.

In another aspect the invention relates to a derivative of a human amylin analogue.

In another aspect of the invention, the derivative has from 1-12 amino acid substitutions compared to human amylin.

In another aspect of the invention, the derivative has from 1-6 amino acid substitutions compared to human amylin.

In another aspect of the invention, the derivative has from 1-5 amino acid substitutions compared to human amylin.

In another aspect of the invention, the derivative has from 1-4 amino acid substitutions compared to human amylin.

In another aspect of the invention, the derivative has from 1-3 amino acid substitutions compared to human amylin.

In another aspect of the invention, the derivative has from 1-2 amino acid substitutions compared to human amylin.

In another aspect the invention relates to a derivative of a human amylin analogue which is further modified according to the invention, wherein the human amylin analogue is 25Pro 28Pro 29Pro human amylin (SEQ ID NO: 1):

Lys-Cys-Asn-Thr-Ala-Thr-Cys-Ala-Thr-Gln-Arg-Leu-Ala-Asn-Phe-Leu-Val-His-Ser-
Ser-Asn-Asn-Phe-Gly-Pro-Ile-Leu-Pro-Pro-Thr-Asn-Val-Gly-Ser-Asn-Thr-Tyr (SEQ ID NO:1).

The invention relates in another aspect to a derivative, wherein the albumin binding residue or the polyethylene glycol polymer, optionally via a linker is linked via the ε-amino group of the lysine residue.

The invention relates in another aspect to a derivative, wherein the albumin binding residue or the polyethylene glycol polymer, optionally via a linker is linked via the alpha-amino group of the lysine residue.

The invention relates in another aspect to a derivative, wherein the lysine residue is linked to an albumin binding residue via a linker.

The term "linked to" as used herein means chemically connected via a covalent bond. For example a lysine residue is linked to an albumin binding residue via a chemical bond. Such a chemical bond can as an example be obtained by derivatisation of an epsilon amino group of lysine by acylation with an active ester of an albumin binding residue such as a long fatty acid.

Other examples of connecting two chemical moieties as used in the present invention includes but is not limited to alkylation, ester formation, amide formation or maleimide coupling.

The term "albumin binding residue" as used herein means in one aspect of the invention a residue which binds non-covalently to human serum albumin. The albumin binding residue connected to the therapeutic polypeptide typically has an affinity (binding constant) below 10 µM to human serum albumin and preferably below 1 µM. A range of albumin binding residues are known among linear and branched lipohophillic moieties containing 4-40 carbon atoms, compounds with a cyclopentanophenanthrene skeleton, peptides having 10-30 amino acid residues etc.

Albumin binding affinity may be determined by several methods known within the art. In one method the derivative to be measured is radiolabeled with e.g. ¹²⁵I or ³H and incubated with immobilized albumin (Kurtzhals et.al., Biochem.J., 312, 725-731 (1995)). The binding of the derivative relative to a standard is calculated. In another method a related compound is radiolabeled and its binding to albumin immobilized on e.g. SPA beads is competed by a dilution series of the derivative to be measured. The EC50 value for the competition is a measure of the affinity of the derivative. In a third method, the receptor affinity or potency of a derivative is measured at different concentrations of albumin, and the shift in relative affinity or potency of the derivative as a function of albumin concentration reflects its affinity for albumin.

In one aspect of the invention the albumin binding residue comprises a group which can be negatively charged.

The term "C₁₋₆₋alkyl" as used herein means a saturated, branched, straight or cyclic hydrocarbon group having from 1 to 6 carbon atoms. Representative examples include, but are not limited to, methyl, ethyl, n-propyl, isopropyl, butyl, isobutyl, *sec*-butyl, *tert*-butyl, n-pentyl, isopentyl, neopentyl, *tert*-pentyl, n-hexyl, isohexyl, cyclohexane and the like.

The term "linker" as used herein means a spacer (the two terms spacer and linker is used interchangeably in the present specification) that separates a peptide and an albumin binding residue or a polyethylene glycol polymer.

The term "hydrophilic linker" as used herein means a spacer that separates a peptide and an albumin binding residue with a chemical moiety which comprises at least 5 heavy atoms where 30-50% of these are either N or O.

In one aspect of the invention, the linker comprises 1 or more negatively charged entities to obtain an amylin derivative according to the invention wherein 0-8 additional charges have been added to the molecule compared to the parent amylin.

In one aspect of the invention, the linker comprises an unbranched alkane α,ω-dicarboxylic acid group having from 1 to 7 methylene groups, preferably two methylene groups which linker forms a bridge between an amino group of the peptide and an amino group of the albumin binding residue or a polyethylene glycol polymer.

In another aspect of the invention, the linker comprises one or more alkylene glycol units, such as 1 to 5 alkylene glycol units. The alkylene glycol units are in a further aspect ethylene glycol, propylene glycol or butylene glycol but can also be higher alkylene glycols.

In another aspect of the invention, the linker is a hydrophilic linker selected from -(CH₂)ₗD[(CH₂)ₙE]ₘ(CH₂)ₚ-Q_{q}-,
wherein
I, m and n independently are 1-20 and p is 0-10,
Q is -Z-(CH₂)ₗD[(CH₂)ₙG]ₘ(CH₂)ₚ-,
q is an integer in the range from 0 to 5,
each D, E, and G are independently selected from -O-, -NR³-, -N(COR⁴)-, -PR⁵(O)-, and -P(OR⁶)(O)-, wherein R³, R⁴, R⁵, and R⁶ independently represent hydrogen or C₁₋₆-alkyl,
Z is selected from -C(O)NH-, -C(O)NHCH₂-, -OC(O)NH -, -C(O)NHCH₂CH₂-, -C(O)CH₂-, -C(O)CH=CH-, -(CH₂)ₛ-, -C(O)-, -C(O)O- or -NHC(O)-, wherein s is 0 or 1.

In another aspect of the invention, the linker is a hydrophilic linker as defined above wherein I is 1 or 2, n and m are independently 1-10 and p is 0-10.

In another aspect of the invention, the linker is a hydrophilic linker as defined above wherein D is -O-.

In another aspect of the invention, the linker is a hydrophilic linker as defined above wherein E is -O-.

In yet another aspect of the invention, the hydrophilic linker is -CH₂O[(CH₂)₂O]ₘ(CH₂)ₚQ_{q}-, wherein m is 1-10, p is 1-3, and Q is -Z-CH₂O[(CH₂)₂O]ₘ(CH₂)ₚ- wherein Z is as defined above.

In another aspect of the invention, the linker is a hydrophilic linker as defined above wherein q is 1.

In another aspect of the invention, the linker is a hydrophilic linker as defined above wherein G is -O-.

In another aspect of the invention, the linker is a hydrophilic linker as defined above wherein Z is selected from the group consisting of -C(O)NH-, -C(O)NHCH₂-, and -OC(O)NH-.

In another aspect of the invention, the linker is a hydrophilic linker as defined above wherein q is 0.

In another aspect of the invention, the linker is a hydrophilic linker as defined above wherein I is 2.

In another aspect of the invention, the linker is a hydrophilic linker as defined above wherein n is 2.

In one aspect of this invention a "hydrophilic linker" B is used that separates a peptide and an albumin binding residue with a chemical moiety.

In one aspect of this invention, the hydrophilic linker B is -C(O)-(CH₂)ₗ-O-[(CH₂CH₂-O]ₘ-(CH₂)ₚ-[NHC(O)-(CH₂)ₗ-O-[(CH₂)ₙ-O]ₘ-(CH₂)ₚ]_{q}-NH-, wherein I, m, n, and p independently are 1-5, and q is 0-5.

In yet another aspect of this invention, the hydrophilic linker B is -C(O)-CH₂-O-CH₂CH₂-O-CH₂CH₂ [NHC(O)-CH₂-O-CH₂CH₂O-CH₂CH₂]_{q}-NH-, wherein q is 0-5.

In yet another aspect of this invention, the hydrophilic linker B is -C(O)-CH₂-O-CH₂CH₂-O-CH₂CH₂ -NHC(O)-CH₂-O-CH₂CH₂O -CH₂CH₂-NH-.

In yet another aspect of the invention, the hydrophilic linker B is -[CH₂CH₂O]ₘ₊₁ (CH₂)ₚQ_{q}-
wherein m and p independently is 0-10, q is 0-5 and
Q is -Z-(CH₂)ₗD[(CH₂)ₙG]ₘ(CH₂)ₚ- as defined above.

In yet another aspect of the invention, the hydrophilic linker B is -(CH₂)ₗ-O-[(CH₂)ₙ-O]ₘ-(CH₂)ₚ-[C(O)NH-(CH₂)ₗ-O-[(CH₂)ₙ-O]ₘ-(CH₂)ₚ]_{q}-,
wherein I, m, n, and p independently are 1-5, and q is 0-5.

In a further aspect of the invention, the linker comprises an amino acid residue except Cys, or a dipeptide such as Gly-Lys, Glu-Glu or Glu-Arg, or a tripeptide such as Gly-Gly-Lys, Glu-Glu-Glu or Glu-Glu-Arg. In the present text, the expression "a dipeptide such as Gly-Lys" is used to designate a dipeptide wherein the C-terminal amino acid residue is Lys, His or Trp, preferably Lys, and wherein the N-terminal amino acid residue is selected from the group comprising Ala, Arg, Asp, Asn, Gly, Glu, Gln, Ile, Leu, Val, Phe and Pro.

Suitable PEG polymers are typically commercially available or may be made by techniques well-known to those skilled in the art.

In one aspect of the invention, the PEG polymer has a molecular weight of greater than 700D, in a further aspect a molecular weight greater than 5 kD, in yet a further aspect greater than 10 kD, and in a even further aspect greater that 20kD. The PEG polymer may be linear or branched. In cases where the PEG polymer is greater than 20KDa, the PEG polymer is preferable having a branched structure, such as for example, a 43 kD branched PEG-peptide (Shearwater 2001 catalog # 2D3XOT01, mPEG2-MAL).

The attachment of a PEG on an intact peptide can be accomplished by attaching the PEG on the opposite side of the peptide surface that interacts with the receptor.

In one aspect of the invention, the attachment of PEG will occur on the amino acid residue in position 2, 3, 4, 5, or 6 of human amylin or an analog thereof which is substituted with a lysine residue or a cysteine, optionally via a linker. In a further aspect of the invention, the attachment of PEG will occur on the amino acid residue in position 23, 24, 25, 26, 27 or 28 of human amylin or an analog thereof which is substituted with a lysine residue or a cysteine, optionally via a linker. In yet a further aspect of the invention, the attachment of PEG will occur on the amino acid residue in position 32, 33, 34, 35, 36, or 37 of human amylin or an analog thereof which is substituted with a lysine residue or a cysteine, optionally via a linker.

There are several strategies for coupling PEG to peptides (see, e.g. Veronese, Biomaterials 22:405-417, 2001), all of which are incorporated herein by reference in their entirety. Those skilled in the art, will therefore be able to utilize well-known techniques for linking the PEG polymer to human amylin or the amylin analogs described herein.

Briefly, cysteine PEGylation is one method for site-specific PEGylation, and can be accomplished by introducing a unique cysteine mutation at one of the specific positions on human amylin or the amylin analog and then reacting the resulting peptide with a cysteine-specific PEGylation reagent, such as PEG-maleimide. It may be necessary to mutate the peptide in order to allow for site-specific PEGylation. For example, if the peptide contains cysteine residues, these will need to be substituted with conservative amino acids in order to ensure site-specific PEGylation. In addition, rigid linkers, including but not limited to "GGS", "GGSGGS", and "PPPS" may be added to the C-terminus, but before the site of PEG attachment (i.e. a unique cysteine residue).

In one aspect of the invention, the albumin binding residue is a lipophilic residue. In a further aspect, the lipophilic residue is connected to a lysine residue optionally via a linker by conjugation chemistry such as by alkylation, acylation, ester formation, or amide formation or to a cysteine residue by maleimide coupling.

In a further aspect of the invention, the albumin binding residue is negatively charged at physiological pH. In another aspect of the invention, the albumin binding residue comprises a group which can be negatively charged. One preferred group which can be negatively charged is a carboxylic acid group.

In a further aspect of the invention, the albumin binding residue binds non-covalently to albumin. In another aspect of the invention, the albumin binding residue has a binding affinity towards human serum albumin better than about 10 µM or better than about 1 µM.

In yet another aspect of the invention, the albumin binding residue is selected from the group consisting of a straight chain alkyl group, a branched alkyl group, a group which has an ω-carboxylic acid group, and a partially or completely hydrogenated cyclopentanophenanthrene skeleton.

In a further aspect of the invention, the albumin binding residue is a cibacronyl residue.

In a further aspect of the invention, the albumin binding residue has from 6 to 40 carbon atoms, from 8 to 26 carbon atoms or from 8 to 20 carbon atoms.

In a further aspect of the invention, the albumin binding residue is an acyl group selected from the group comprising CH₃(CH₂)ᵣCO-, wherein r is an integer from 4 to 38, preferably an integer from 4 to 24, more preferred selected from the group comprising CH₃(CH₂)₆CO-, CH₃(CH₂)₈CO-, CH₃(CH₂)₁₀CO-, CH₃(CH₂)₁₂CO-, CH₃(CH₂)₁₄CO-, CH₃(CH₂)₁₆CO-, CH₃(CH₂)₁₈CO-, CH₃(CH₂)₂₀CO- and CH₃(CH₂)₂₂CO-.

In another aspect of the invention, the albumin binding residue is an acyl group of a straight-chain or branched alkane α,ω-dicarboxylic acid.

In another aspect of the invention, the albumin binding residue is an acyl group selected from the group comprising HOOC(CH₂)ₛCO-, wherein s is an integer from 4 to 38, preferably an integer from 4 to 24, more preferred selected from the group comprising HOOC(CH₂)₁₄CO-, HOOC(CH₂)₁₆CO-, HOOC(CH₂)₁₈CO-, HOOC(CH₂)₂₀CO- and HOOC(CH₂)₂₂CO-.

In another aspect of the invention, the albumin binding residue is a group of the formula CH₃(CH₂)ᵥCO-NHCH(COOH)(CH₂)₂CO-, wherein v is an integer from 10 to 24.

In another aspect of the invention, the albumin binding residue is a group of the formula CH₃(CH₂)_{w}CO-NHCH((CH₂)₂COOH)CO-, wherein w is an integer from 8 to 24.

In another aspect of the invention, the albumin binding residue is a group of the formula COOH(CH₂)ₓCO- wherein x is an integer from 8 to 24.

In another aspect of the invention, the albumin binding residue is a group of the formula -NHCH(COOH)(CH₂)₄NH-CO(CH₂)_{y}CH₃, wherein y is an integer from 8 to 18.

In one aspect of this invention, the combined albumin binding residue and linker is -C(O)-(CH₂)ₗ-O-[(CH₂CH₂-O]ₘ-(CH₂)ₚ-[NHC(O)-(CH₂)ₗ-O-[(CH₂)ₙ-O]ₘ-(CH₂)ₚ]_{q}-NH-W,
wherein I, m, n, and p independently are 1-5, and q is 0-5
and W is selected from the group consisting of CH₃(CH₂)₆CO-, CH₃(CH₂)₈CO-, CH₃(CH₂)₁₀CO-, CH₃(CH₂)₁₂CO-, CH₃(CH₂)₁₄CO-, CH₃(CH₂)₁₆CO-, CH₃(CH₂)₁₈CO-, CH₃(CH₂)₂₀CO- , CH₃(CH₂)₂₂CO-, HOOC(CH₂)₁₄CO-, HOOC(CH₂)₁₆CO-, HOOC(CH₂)₁₈CO-, HOOC(CH₂)₂₀CO- and HOOC(CH₂)₂₂CO-.

In one aspect of this invention, the combined albumin binding residue and linker is -C(O)-CH₂-O-CH₂CH₂-O-CH₂CH₂ [NHC(O)-CH₂-O-CH₂CH₂-O-CH₂CH₂]_{q}-NH-Ac,
wherein q is 0-5 and wherein Ac is acetyl.

In another aspect of this invention, the combined albumin binding residue and linker is a group of the formula -C(O)-CH₂-O-CH₂CH₂-O-CH₂CH₂ -NHC(O)-CH₂O-CH₂CH₂O -CH₂CH₂-NH-Ac,

wherein Ac is acetyl.

In another aspect of this invention, the combined albumin binding residue and linker is 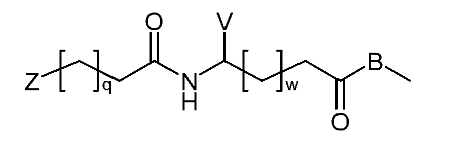 wherein B is defined as described above, and Z, V, q and w is defined as below.

In another aspect of this invention, the combined albumin binding residue and linker is 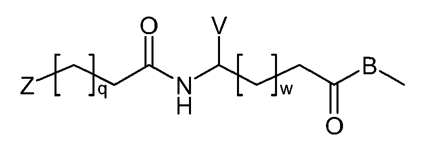
wherein Z is CH₃ or COOH, V is H or COOH, q is 7 to 21, w is 0 to 5, and k is 0 to 5
and B is -C(O)-(CH₂)ₗ-O-[(CH₂CH₂-O]ₘ-(CH₂)ₚ-[NHC(O)-(CH₂)ₗ-O-[(CH₂)ₙ-O]ₘ-(CH₂)ₚ]_{q}-NH-,
wherein I, m, n, and p independently are 1-5, and q is 0-5.

In another aspect of this invention, the combined albumin binding residue and linker is 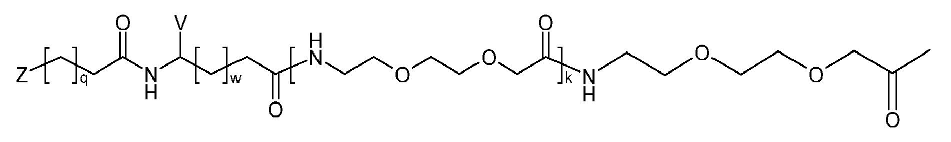 wherein Z is CH₃ or COOH, V is H or COOH, q is 7 to 21, w is 0 to 5, and k is 0 to 5. In another aspect of this invention, Z is CH₃ and V is COOH. In another aspect of this invention V is H and Z is COOH. In another aspect of this invention, V is H and Z is CH₃,. In another aspect of this invention, V and Z are both COOH. In a preferred aspect of this invention, q is 13 to 19, and more preferable q is 13 to 15. In a preferred aspect of this invention, k is 1 to 4, and more preferable 1 to 2. In a preferred aspect of this invention, w is 1 to 4, and more preferable 1 to 2.

In another aspect of this invention, the combined albumin binding residue and linker is 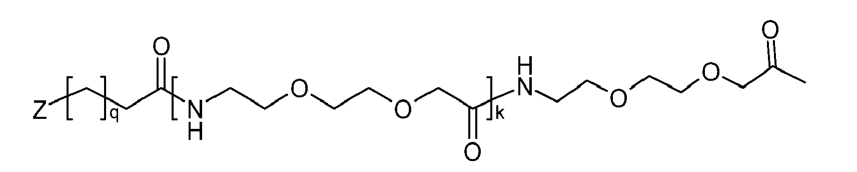 wherein Z is CH₃ or COOH, q is 7 to 21, and k is 0 to 5. In another aspect of this invention, Z is CH₃. In another aspect of this invention, Z is COOH. In a preferred aspect of this invention, q is 13 to 19, and more preferable q is 13, 14 or 15. In a preferred aspect of this invention, k is 1 to 4, and more preferable 1 or 2.

In another aspect of the invention, the albumin binding residue is a peptide, such as a peptide comprising less than 40 amino acid residues. A number of small peptides which are albumin binding residues as well as a method for their identification is found in J. Biol Chem. 277, 38 (2002) 35035-35043.

In another aspect of the invention, the albumin binding residue optionally via a linker is connected via the ε-amino group of a lysine residue.

In a further aspect, the present invention relates to a amylin derivative wherein a lipophilic residue is connected to the parent peptide by means of a linker which is an amino acid residue except Cys, or is a dipeptide such as Gly-Lys, Glu-Arg or Glu-Glu, or is a tripeptide such as Gly-Gly-Lys, Glu-Glu-Arg or Glu-Glu-Glu or is a multipeptide consisting of 4-10 amino acids, and wherein a carboxyl group of the parent peptide forms an amide bond with an amino group of a Lys residue or a dipeptide, tripeptide or multipeptide containing a Lys residue, and the other amino group of the Lys residue or a dipeptide, tripeptide or muitpeptide containing a Lys residue forms an amide bond with a carboxyl group of the lipophilic residue.

In a further aspect, the present invention relates to a amylin derivative wherein a lipophilic residue is connected to the parent peptide by means of a linker which is an amino acid residue except Cys, or is a dipeptide such as Gly-Lys, Glu-Arg or Glu-Glu, or is a tripeptide such as Gly-Gly-Lys, Glu-Glu-Arg or Glu-Glu-Glu, or is a multipeptide consisting of 4-10 amino acids, and wherein an amino group of the parent peptide forms an amide bond with a carboxylic group of the amino acid residue or dipeptide, tripeptide or multipeptide linker, and an amino group of the amino acid residue or dipeptide, tripeptide or multipeptide linker forms an amide bond with a carboxyl group of the lipophilic residue.

In a further aspect, the present invention relates to a amylin derivative wherein a lipophilic residue is connected to the parent peptide by means of a linker which is an amino acid residue except Cys, or is a dipeptide such as Gly-Lys, Glu-Arg or Glu-Glu, or is a tripeptide such as Gly-Gly-Lys, Glu-Glu-Arg or Glu-Glu-Glu, or is a multipeptide consisting of 4-10 amino acids, and wherein a carboxyl group of the parent peptide forms an amide bond with an amino group of the amino acid residue linker or dipeptide, tripeptide or multipeptide linker, and the carboxyl group of the amino acid residue linker or dipeptide, tripeptide or multipeptide linker forms an amide bond with an amino group of the lipophilic residue.

In a further aspect, the present invention relates to a amylin derivative wherein a lipophilic residue is connected to the parent peptide by means of a linker which is an amino acid residue except Cys, or is a dipeptide such as Gly-Lys, Glu-Arg or Glu-Glu, or is a tripeptide such as Gly-Gly-Lys, Glu-Glu-Arg or Glu-Glu-Glu, or is a multipeptide consisting of 4-10 amino acids, and wherein a carboxyl group of the parent peptide forms an amide bond with an amino group of a linker which is Asp or Glu, or a dipeptide, tripeptide or multipeptide linker containing an Asp or Glu residue, and a carboxyl group of the linker forms an amide bond with an amino group of the lipophilic residue.

In one aspect of the invention, the C-terminal of the amylin derivative may be terminated as either an acid or amide. In a preferred aspect, the C-terminal of the amylin derivative is an amide.

In a further aspect, the present invention relates to an amylin derivative wherein a lipophilic residue is connected to the parent peptide by means of a N-terminal extension containing a lysine residue which is Lys, or is a dipeptide such as Gly-Lys, or is a tripeptide such as Gly-Gly-Lys, or is a multipeptide consisting of 4-10 amino acids having a Lys in the N-terminal position, and wherein an amino group of the Lys residue or the N-terminal Lys residue of the dipeptide, tripeptide or muitpeptide containing a Lys residue forms an amide bond with a carboxyl group of the lipophilic residue. In one aspect the amide bond is between the ε-amino group of the lysine residue and the lipophilic residue. In one aspect the amide bond is between the α-amino group of the lysine residue and the lipophilic residue.

In a further aspect, the present invention relates to a amylin derivative wherein a lipophilic residue is connected to the parent peptide by means of a N-terminal extension which is an amino acid residue, or is a dipeptide such as Gly-Lys, Glu-Arg or Glu-Glu, or is a tripeptide such as Gly-Gly-Lys, Glu-Glu-Arg or Glu-Glu-Glu, or is a multipeptide consisting of 4-10 amino acids, and wherein an amino group of the N-terminal amino acid of said N-terminal extension forms an amide bond with a carboxyl group of the lipophilic residue.

In a further aspect, the present invention relates to a amylin derivative wherein a lipophilic residue is connected to the parent peptide by means of a N-terminal extension which is an amino acid residue, or is a dipeptide such as Gly-Lys, Glu-Arg or Glu-Glu, or is a tripeptide such as Gly-Gly-Lys, Glu-Glu-Arg or Glu-Glu-Glu, or is a multipeptide consisting of 4-10 amino acids, and wherein a carboxyl group of the N-terminal amino acid of said N-terminal extension forms an amide bond with an amino group of the lipophilic residue.

In one aspect of the invention, the amino acid sequence of the amylin derivative is selected from the group consisting of:
KCNTATCATQRLANFLVHSSNNFGPILPPTNVGSNTY (SEQ ID No. 1),
KCKTATCATQRLANFLVHSSNNFGPILPPTNVGSNTY (SEQ ID No. 2),
KCNTATCATQRLANFLVHSSNNFGPILPKTNVGSNTY (SEQ ID No. 3),
KCNTATCATQRLANFLVHSSKNFGPILPPTNVGSNTY (SEQ ID No. 4),
KCNTATCATQRLANFLKHSSNNFGPILPPTNVGSNTY (SEQ ID No. 5),
KCNTATCATQRLANFLVHSSNNFGKILPPTNVGSNTY (SEQ ID No. 6),
KCNTATCATQRLANFLVRSSNNFGKILPPTNVGSNTY (SEQ ID No. 7),
CNTATCATQRLANFLVRSSNNLGPVLPPTNVGSNTY (SEQ ID No. 8),
KCKTATCATQRLANFLVPSSNNFGPILPPTNVGSNTY (SEQ ID No. 9),
KCNTATCATQRLANFLVRSSNNFGPILPPTNVGSNTY (SEQ ID No. 10),
KCKTATCATQRLANFLVRSSNNFGPILPPTNVGSNTY (SEQ ID No. 11),
KCGTATCATQRLANFLARSSNNFGPILSPTNVGSNTY (SEQ ID No. 12),
EERECNTATCATQRLANFLVHSSNNFGPILPPTNVGSNTY (SEQ ID No. 13),
KCKTATCATQRLANFLVRSSQNLGPVLPPTNVGSNTY (SEQ ID No. 14),
ECKTATCATQRLANFLVHSSNNFGPILPPTNVGSNTY (SEQ ID No. 15),
EECNTATCATQRLANFLVHSSNNFGPILPPTNVGSNTY (SEQ ID No. 16).
wherein at least one of the amino acid residue(s) or is linked to an albumin binding residue or a polyethylene glycol polymer, optionally via a linker.

In a further aspect, only one lysine residue in an amylin derivative is linked to an albumin binding residue or a polyethylene glycol polymer, optionally via a linker.

In a preferred aspect, the C-terminal of the amylin derivatives having any one of the amino acid sequences of SEQ ID No 1 to 16 is an amide.

In a further aspect of the invention, the albumin binding residue combined with a linker is selected from the group consisting of: 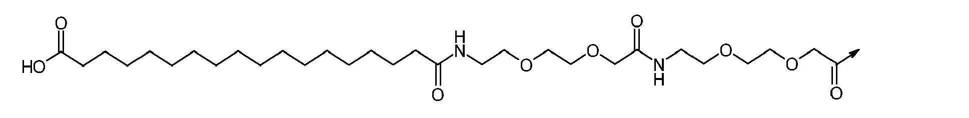 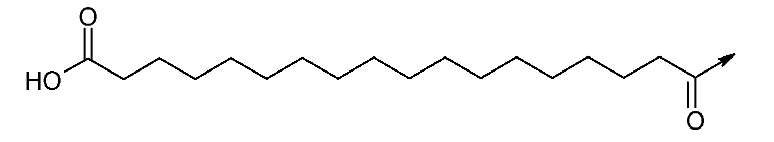 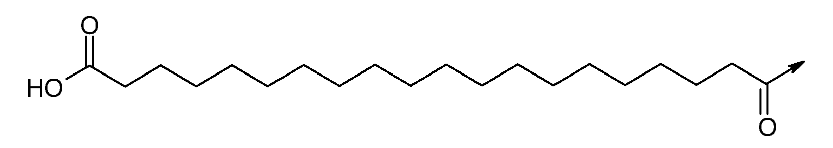 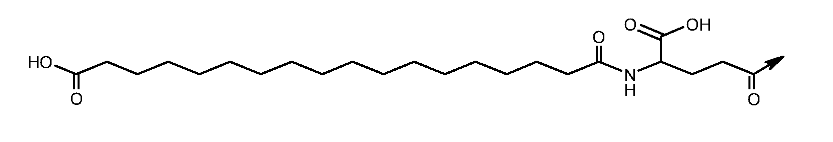 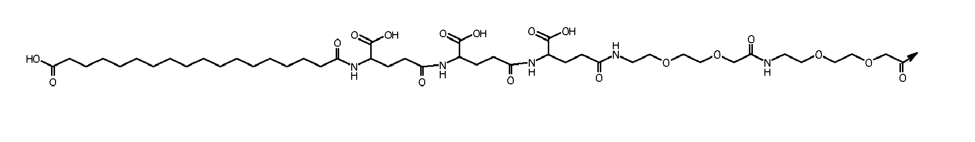 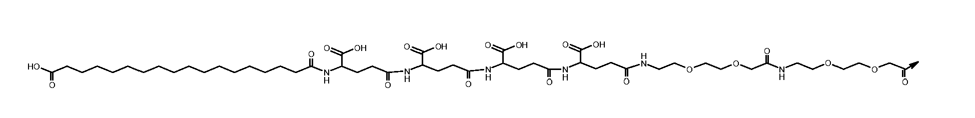 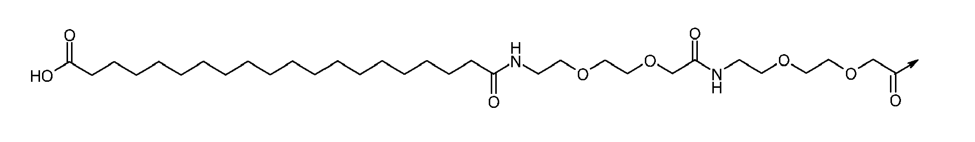 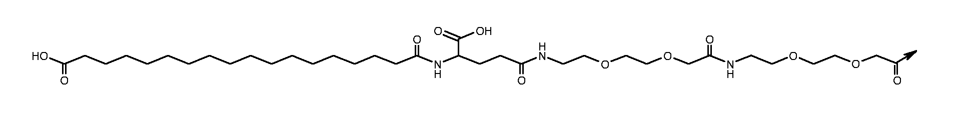 and

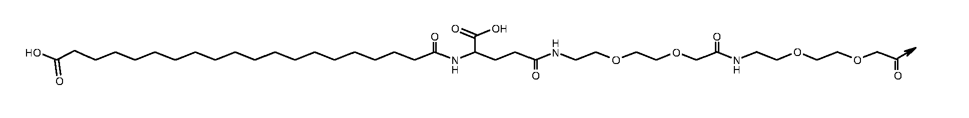

In one aspect of the invention, the amylin derivative is selected from the group consisting of: KCKTATCATQRLANFLVHSSNNFGPILPPTNVGSNTY (SEQ ID No 2), wherein the lysine residue in position 3 is linked to an albumin binding residue combined with a linker with a formula selected from the group consisting of: 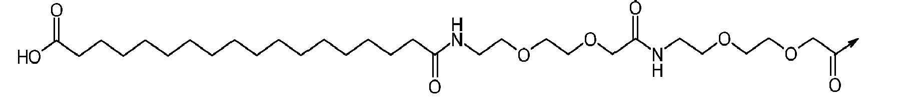 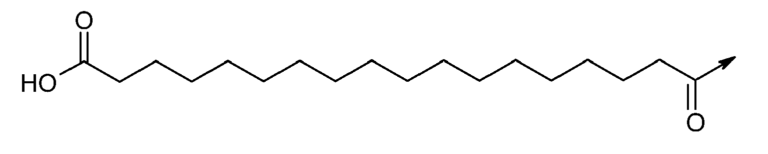 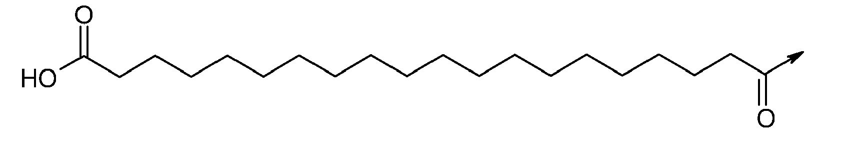 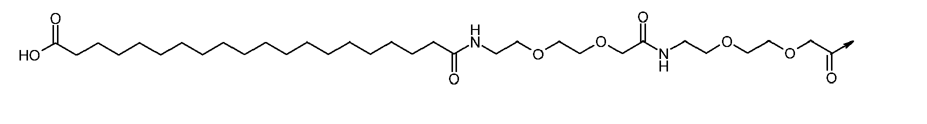 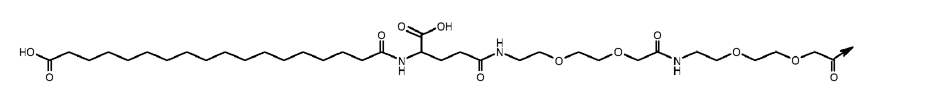 and 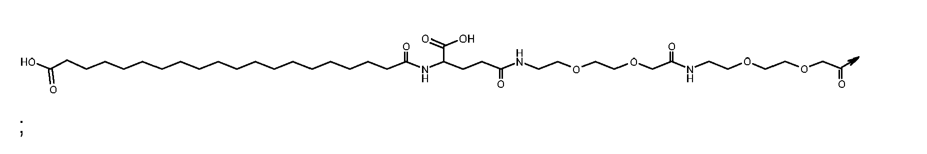 KCNTATCATQRLANFLVHSSNNFGPILPKTNVGSNTY (SEQ ID No 3), wherein the lysine residue in position 29 is linked to an albumin binding residue combined with a linker with a formula selected from the group consisting of: 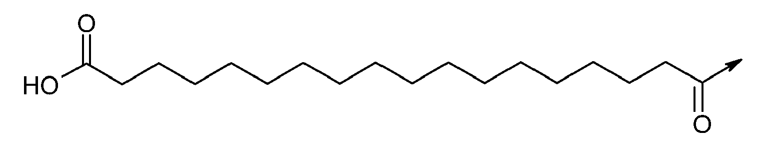 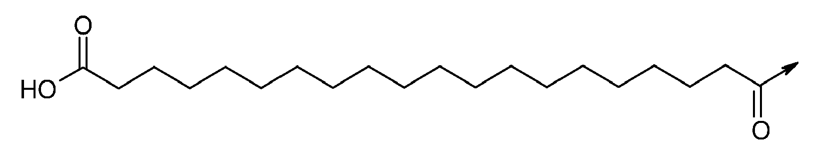 and 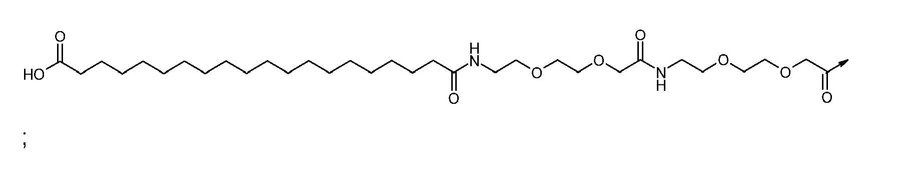 KCNTATCATQRLANFLVHSSKNFGPILPPTNVGSNTY (SEQ ID No 4), wherein the lysine residue in position 21 is linked to an albumin binding residue combined with a linker with the formula: 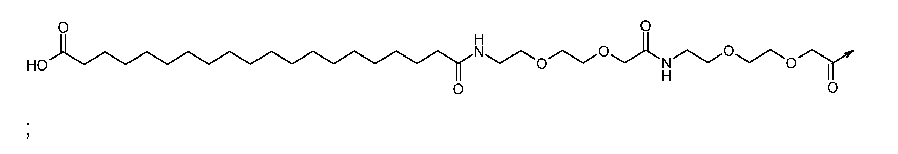 KCNTATCATQRLANFLKHSSNNFGPILPPTNVGSNTY (SEQ ID No 5), wherein the lysine residue in position 17 is linked to an albumin binding residue combined with a linker with the formula: 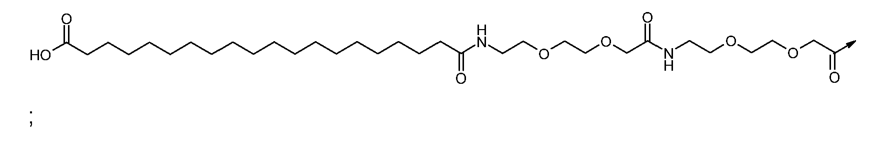 KCNTATCATQRLANFLVHSSNNFGKILPPTNVGSNTY (SEQ ID No 6), wherein the lysine residue in position 25 is linked to an albumin binding residue combined with a linker with a formula selected from the group consisting of: 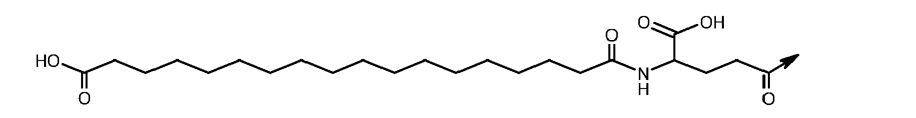 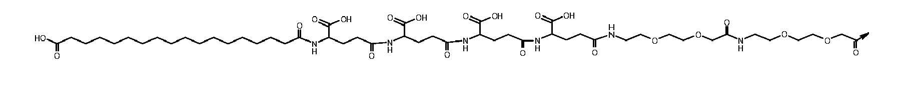 and 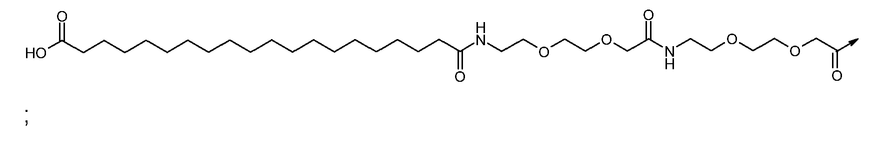 KCNTATCATQRLANFLVRSSNNFGKILPPTNVGSNTY (SEQ ID No 7), wherein the lysine residue in position 25 is linked to an albumin binding residue combined with a linker with the formula: 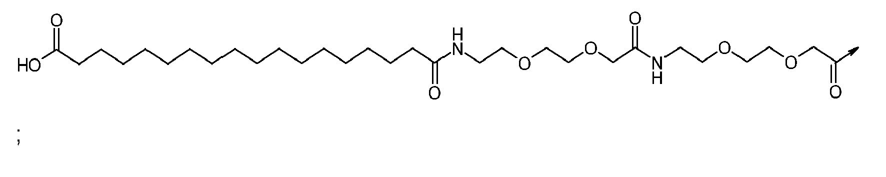 CNTATCATQRLANFLVRSSNNLGPVLPPTNVGSNTY (SEQ ID No 8), wherein the cysteine residue in position 1 is linked to an albumin binding residue combined with a linker with a formula selected from the group consisting of: 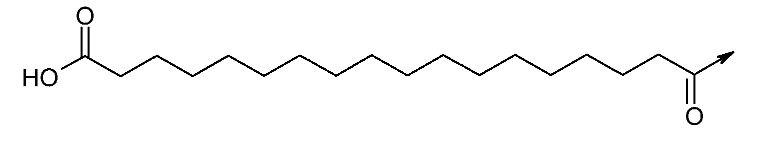 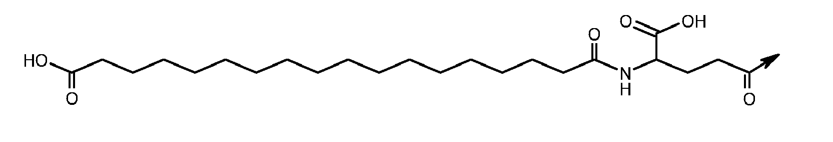 and 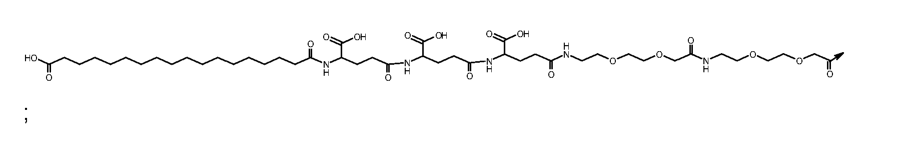 KCKTATCATQRLANFLVPSSNNFGPILPPTNVGSNTY (SEQ ID No 9), wherein the lysine residue in position 3 is linked to an albumin binding residue combined with a linker with a formula selected from the group consisting of: 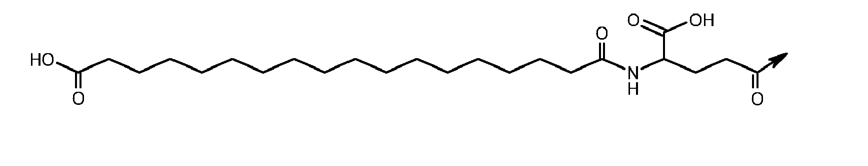 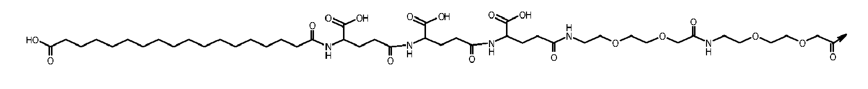 and 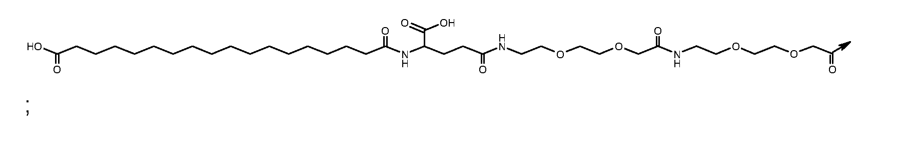 KCNTATCATQRLANFLVRSSNNFGPILPPTNVGSNTY (SEQ ID No 10), wherein the lysine residue in position 1 is linked to an albumin binding residue combined with a linker with a formula selected from the group consisting of: 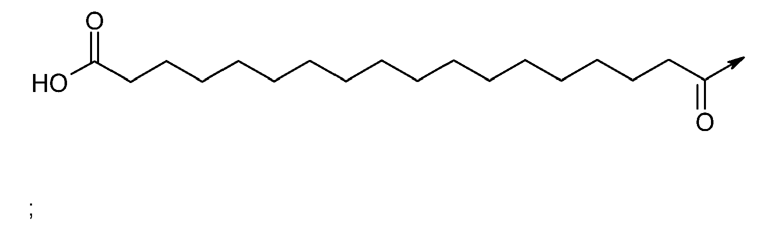 KCKTATCATQRLANFLVRSSNNFGPILPPTNVGSNTY (SEQ ID No 11), wherein the lysine residue in position 3 is linked to an albumin binding residue combined with a linker with a formula selected from the group consisting of: 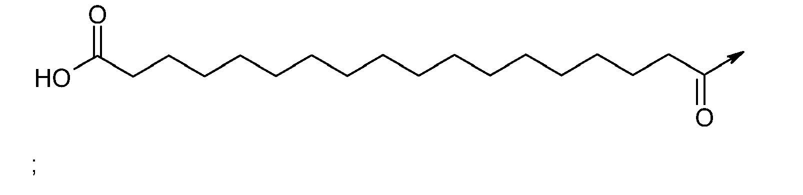 KCGTATCATQRLANFLARSSNNFGPILSPTNVGSNTY (SEQ ID No 12), wherein the glycine residue in position 3 is linked to an albumin binding residue combined with a linker with a formula selected from the group consisting of: 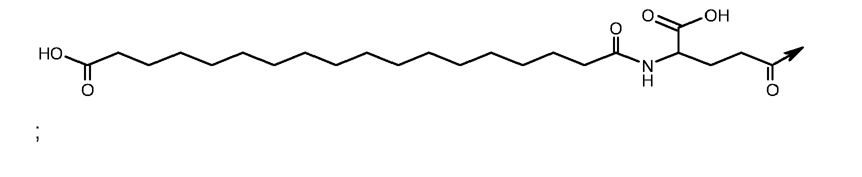 EERECNTATCATQRLANFLVHSSNNFGPILPPTNVGSNTY (SEQ ID No 13), wherein the glutamic acid residue in position 1 is linked to an albumin binding residue combined with a linker with a formula selected from the group consisting of: 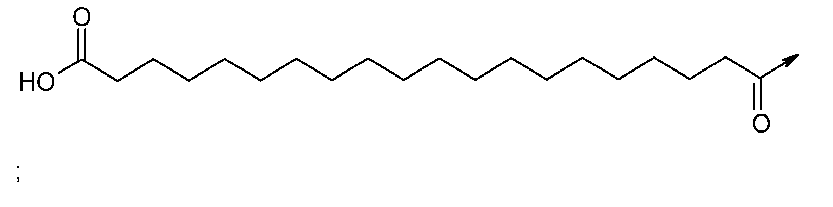 KCKTATCATQRLANFLVRSSQNLGPVLPPTNVGSNTY (SEQ ID No 14), wherein the lysine residue in position 3 is linked to an albumin binding residue combined with a linker with a formula selected from the group consisting of: 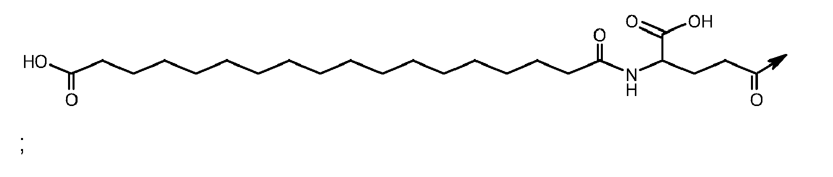 ECKTATCATQRLANFLVHSSNNFGPILPPTNVGSNTY (SEQ ID No 15), wherein the lysine residue in position 3 is linked to an albumin binding residue combined with a linker with a formula selected from the group consisting of: 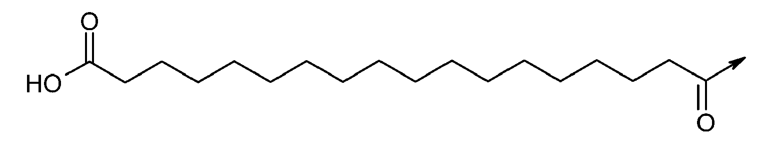 and
EECNTATCATQRLANFLVHSSNNFGPILPPTNVGSNTY (SEQ ID No 16), wherein the glutamic acid residue in position 1 is linked to an albumin binding residue combined with a linker with a formula selected from the group consisting of:

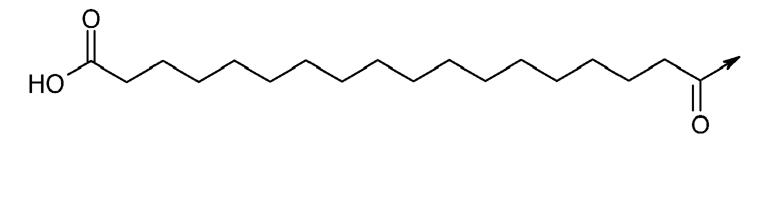

The invention relates in a further aspect to a method for increasing the time of action in a patient of human amylin or an analogue thereof, characterized by
a) modifying the amino acid residue in position 1 by optionally substituting with any natural amino acid, and by connecting said natural amino acid to a N-terminal extension consisting of 1-10 amino acids, and further
   a. linking said extension to an albumin binding residue or a polyethylene glycol polymer, or
   b. modifying an amino acid residue in position 2-37 by substitution with a lysine residue, and by linking said lysine residue to an albumin binding residue or a polyethylene glycol polymer, optionally via a linker;
   or
b) modifying the amino acid residue in position 3 by substitution with a lysine residue and by linking said lysine residue to an albumin binding residue or a polyethylene glycol polymer, optionally via a linker, and optionally modifying the amino acid in position 1 by substitution with a glutamine residue;
   or
c) modifying an amino acid residue in position 21, 25 or 29 by substitution with a lysine residue and by linking said lysine residue to an albumin binding residue or a polyethylene glycol polymer, optionally via a linker;
   or
d) modifying the amino acid residue in position 18 by substitution with an arginine residue, and further modifying an amino acid residue in position 1-17 or 19-37 by substitution with a lysine residue, and by linking to said lysine residue an albumin binding residue or a polyethylene glycol polymer, optionally via a linker;
wherein the amino acid numbering conforms with the amino acid numbering in SEQ ID NO:17.

The invention relates in a further aspect to a method for increasing the time of action in a patient of human amylin or an analogue thereof, characterized by modifying
a) the amino acid residue in position 1 by optionally substituting with any natural amino acid, and by connecting said natural amino acid to a N-terminal extension consisting of 1-10 amino acids, and further
   a. linking said extension to an albumin binding residue or a polyethylene glycol polymer, or
   b. modifying an amino acid residue in position 2-37 by substitution with a lysine residue, and by linking said lysine residue to an albumin binding residue or a polyethylene glycol polymer, optionally via a linker;
   or
b) the amino acid residue in position 3 by substitution with a lysine residue and by linking said lysine residue to an albumin binding residue or a polyethylene glycol polymer, optionally via a linker;
   or
c) an amino acid residue in position 21, 25 or 29 by substitution with a lysine residue and by linking said lysine residue to an albumin binding residue or a polyethylene glycol polymer, optionally via a linker;
   or
d) the amino acid residue in position 18 by substitution with an arginine residue, and further modifying an amino acid residue in position 1-17 or 19-37 by substitution with a lysine residue, and by linking to said lysine residue an albumin binding residue or a polyethylene glycol polymer, optionally via a linker;
wherein the amino acid numbering conforms with the amino acid numbering in SEQ ID NO:17.

In another aspect, the invention relates to a method wherein the time of action in a patient of human amylin or an analogue thereof is increased to more than about 40 hours.

The production of peptides such as human amylin or analogues thereof is well known in the art. The peptides of the invention can thus be produced by classical peptide synthesis, e.g. solid phase peptide synthesis using t-Boc or Fmoc chemistry or other well established techniques, see e.g. Greene and Wuts, "Protective Groups in Organic Synthesis", John Wiley & Sons, 1999. The peptides may also be produced by a method which comprises culturing a host cell containing a DNA sequence encoding the polypeptide and capable of expressing the polypeptide in a suitable nutrient medium under conditions permitting the expression of the peptide. For peptides comprising non-natural amino acid residues, the recombinant cell should be modified such that the non-natural amino acids are incorporated into the peptide, for instance by use of tRNA mutants.

### PHARMACEUTICAL COMPOSITIONS

Pharmaceutical compositions containing a derivative according to the present invention may be prepared by conventional techniques, e.g. as described in Remington's Pharmaceutical Sciences, 1985 or in Remington: The Science and Practice of Pharmacy, 19th edition, 1995.

One object of the present invention is to provide a pharmaceutical formulation comprising a peptide according to the present invention. In one aspect, the peptide is present in the formulation at a concentration of from about 0.1 mg/ml to about 25 mg/ml. In another aspect, the peptide is present in the formulation at a concentration of from about 1 mg/ml to about 10 mg/ml.

In another aspect, the formulation has a pH from 4.0 to 10.0.

In another aspect, the formulation has a pH from 4.0 to 7.0.

In yet another aspect, the formulation has a pH from 7.0 to 10.0.

In yet another aspect, the formulation has a pH from 7.0 to 8.0.

The formulation may further comprise a buffer system, preservative(s), isotonicity agent(s), chelating agent(s), stabilizers and/or surfactants. The use of such excipients in pharmaceutical compositions is well-known to the skilled person. For convenience reference is made to Remington: The Science and Practice of Pharmacy, 19th edition, 1995.

In one aspect of the invention the pharmaceutical formulation is an aqueous formulation, i.e. formulation comprising water. Such formulation is typically a solution or a suspension. In a further aspect of the invention the pharmaceutical formulation is an aqueous solution. The term "aqueous formulation" is defined as a formulation comprising at least 50 %w/w water. Likewise, the term "aqueous solution" is defined as a solution comprising at least 50 %w/w water, and the term "aqueous suspension" is defined as a suspension comprising at least 50 %w/w water.

In another aspect the pharmaceutical formulation is a freeze-dried formulation, whereto the physician or the patient adds solvents and/or diluents prior to use.

In another aspect the pharmaceutical formulation is a dried formulation (e.g. freeze-dried or spray-dried) ready for use without any prior dissolution.

In a further aspect of the invention the buffer is selected from the group consisting of sodium acetate, sodium carbonate, citrate, glycylglycine, histidine, glycine, lysine, arginine, sodium dihydrogen phosphate, disodium hydrogen phosphate, sodium phosphate, and tris(hydroxymethyl)-aminomethan, bicine, tricine, malic acid, succinate, maleic acid, fumaric acid, tartaric acid, aspartic acid or mixtures thereof. Each one of these specific buffers constitutes an alternative aspect of the invention.

In a further aspect of the invention the formulation further comprises a pharmaceutically acceptable preservative. In a further aspect of the invention the formulation further comprises an isotonic agent. In a further aspect of the invention the formulation further comprises a chelating agent.

In a further aspect of the invention the formulation further comprises a stabilizer. The use of a stabilizer in pharmaceutical compositions is well-known to the skilled person. For convenience reference is made to Remington: The Science and Practice of Pharmacy, 19th edition, 1995.

More particularly, compositions of the invention are stabilized liquid pharmaceutical compositions whose therapeutically active components include a polypeptide that possibly exhibits aggregate formation during storage in liquid pharmaceutical formulations. By "aggregate formation" is intended a physical interaction between the polypeptide molecules that results in formation of oligomers, which may remain soluble, or large visible aggregates that precipitate from the solution. By "during storage" is intended a liquid pharmaceutical composition or formulation once prepared, is not immediately administered to a subject. Rather, following preparation, it is packaged for storage, either in a liquid form, in a frozen state, or in a dried form for later reconstitution into a liquid form or other form suitable for administration to a subject. By "dried form" is intended the liquid pharmaceutical composition or formulation is dried either by freeze drying (i.e., lyophilization; see, for example, Williams and Polli (1984) J. Parenteral Sci. Technol. 38:48-59), spray drying (see Masters (1991) in Spray-Drying Handbook (5th ed; Longman Scientific and Technical, Essez, U.K.), pp. 491-676; Broadhead et al. (1992) Drug Devel. Ind. Pharm. 18:1169-1206; and Mumenthaler et al. (1994) Pharm. Res. 11:12-20), or air drying (Carpenter and Crowe (1988) Cryobiology 25:459-470; and Roser (1991) Biopharm. 4:47-53). Aggregate formation by a polypeptide during storage of a liquid pharmaceutical composition can adversely affect biological activity of that polypeptide, resulting in loss of therapeutic efficacy of the pharmaceutical composition. Furthermore, aggregate formation may cause other problems such as blockage of tubing, membranes, or pumps when the polypeptide-containing pharmaceutical composition is administered using an infusion system.

The pharmaceutical compositions of the invention may further comprise an amount of an amino acid base sufficient to decrease aggregate formation by the polypeptide during storage of the composition. By "amino acid base" is intended an amino acid or a combination of amino acids, where any given amino acid is present either in its free base form or in its salt form. Where a combination of amino acids is used, all of the amino acids may be present in their free base forms, all may be present in their salt forms, or some may be present in their free base forms while others are present in their salt forms. In one aspect, amino acids to use in preparing the compositions of the invention are those carrying a charged side chain, such as arginine, lysine, aspartic acid, and glutamic acid. Any stereoisomer (i.e., L, D, or a mixture thereof) of a particular amino acid (e.g. methionine, histidine, imidazole, arginine, lysine, isoleucine, aspartic acid, tryptophan, threonine and mixtures thereof) or combinations of these stereoisomers, may be present in the pharmaceutical compositions of the invention so long as the particular amino acid is present either in its free base form or its salt form. In one aspect the L-stereoisomer is used. Compositions of the invention may also be formulated with analogues of these amino acids. By "amino acid analogue" is intended a derivative of the naturally occurring amino acid that brings about the desired effect of decreasing aggregate formation by the polypeptide during storage of the liquid pharmaceutical compositions of the invention. Suitable arginine analogues include, for example, aminoguanidine, ornithine and N-monoethyl L-arginine, suitable methionine analogues include ethionine and buthionine and suitable cysteine analogues include S-methyl-L cysteine. As with the other amino acids, the amino acid analogues are incorporated into the compositions in either their free base form or their salt form. In a further aspect of the invention the amino acids or amino acid analogues are used in a concentration, which is sufficient to prevent or delay aggregation of the protein.

The pharmaceutical compositions may also comprise additional stabilizing agents, which further enhance stability of a therapeutically active polypeptide therein. The use of a stabilizing agents in pharmaceutical compositions is well-known to the skilled person. For convenience reference is made to Remington: The Science and Practice of Pharmacy, 19th edition, 1995.

In a further aspect of the invention the formulation further comprises a surfactant. In a further aspect of the invention the formulation further comprises protease inhibitors. The use of a protease inhibitor is particular useful in pharmaceutical compositions comprising zymogens of proteases in order to inhibit autocatalysis.

It is possible that other ingredients may be present in the peptide pharmaceutical formulation of the present invention. Such additional ingredients may include wetting agents, emulsifiers, antioxidants, bulking agents, tonicity modifiers, chelating agents, metal ions, oleaginous vehicles, proteins (e.g., human serum albumin, gelatine or proteins) and a zwitterion (e.g., an amino acid such as betaine, taurine, arginine, glycine, lysine and histidine). Such additional ingredients, of course, should not adversely affect the overall stability of the pharmaceutical formulation of the present invention.

Pharmaceutical compositions containing a derivative of human amylin or an analogue thereof according to the present invention may be administered to a patient in need of such treatment at several sites, for example, at topical sites, for example, skin and mucosal sites, at sites which bypass absorption, for example, administration in an artery, in a vein, in the heart, and at sites which involve absorption, for example, administration in the skin, under the skin, in a muscle or in the abdomen.

Administration of pharmaceutical compositions according to the invention may be through several routes of administration, for example, lingual, sublingual, buccal, in the mouth, oral, in the stomach and intestine, nasal, pulmonary, for example, through the bronchioles and alveoli or a combination thereof, epidermal, dermal, transdermal, vaginal, rectal, ocular, for examples through the conjunctiva, uretal, and parenteral to patients in need of such a treatment.

Compositions of the current invention may be administered in several dosage forms, for example, as solutions, suspensions, emulsions, microemulsions, multiple emulsion, foams, salves, pastes, plasters, ointments, tablets, coated tablets, rinses, capsules, for example, hard gelatine capsules and soft gelatine capsules, suppositories, rectal capsules, drops, gels, sprays, powder, aerosols, inhalants, eye drops, ophthalmic ointments, ophthalmic rinses, vaginal pessaries, vaginal rings, vaginal ointments, injection solution, in situ transforming solutions, for example in situ gelling, in situ setting, in situ precipitating, in situ crystallization, infusion solution, and implants.

Compositions of the invention may further be compounded in, or attached to, for example through covalent, hydrophobic and electrostatic interactions, a drug carrier, drug delivery system and advanced drug delivery system in order to further enhance stability of the derivative of human amylin or an analogue thereof increase bioavailability, increase solubility, decrease adverse effects, achieve chronotherapy well known to those skilled in the art, and increase patient compliance or any combination thereof.

Compositions of the current invention are useful in the formulation of solids, semisolids, powder and solutions for pulmonary administration of the derivative of human amylin or an analogue thereof, using, for example a metered dose inhaler, dry powder inhaler and a nebulizer, all being devices well known to those skilled in the art.

Compositions of the current invention are useful in the formulation of controlled, sustained, protracting, retarded, and slow release drug delivery systems.

Parenteral administration may be performed by subcutaneous, intramuscular, intraperitoneal or intravenous injection by means of a syringe, optionally a pen-like syringe. Alternatively, parenteral administration can be performed by means of an infusion pump. A further option is a composition which may be a solution or suspension for the administration of the derivative of human amylin or an analogue thereof in the form of a nasal or pulmonal spray. As a still further option, the pharmaceutical compositions containing the derivative of human amylin or an analogue thereof of the invention can also be adapted to transdermal administration, *e.g.* by needle-free injection or from a patch, optionally an iontophoretic patch, or transmucosal, *e.g.* buccal, administration.

The derivative of human amylin or an analogue thereof can be administered via the pulmonary route in a vehicle, as a solution, suspension or dry powder using any of known types of devices suitable for pulmonary drug delivery. Examples of these comprise of, but are not limited to, the three general types of aerosol-generating for pulmonary drug delivery, and may include jet or ultrasonic nebulizers, metered-dose inhalers, or dry powder inhalers (Cf. Yu J, Chien YW. Pulmonary drug delivery: Physiologic and mechanistic aspects. Crit Rev Ther Drug Carr Sys 14(4) (1997) 395-453).

The term "stabilized formulation" refers to a formulation with increased physical stability, increased chemical stability or increased physical and chemical stability.

The term "physical stability" of the protein formulation as used herein refers to the tendency of the protein to form biologically inactive and/or insoluble aggregates of the protein as a result of exposure of the protein to thermo-mechanical stresses and/or interaction with interfaces and surfaces that are destabilizing, such as hydrophobic surfaces and interfaces. Physical stability of the aqueous protein formulations is evaluated by means of visual inspection and/or turbidity measurements after exposing the formulation filled in suitable containers (e.g. cartridges or vials) to mechanical/physical stress (e.g. agitation) at different temperatures for various time periods. Visual inspection of the formulations is performed in a sharp focused light with a dark background. The turbidity of the formulation is characterized by a visual score ranking the degree of turbidity for instance on a scale from 0 to 3 (a formulation showing no turbidity corresponds to a visual score 0, and a formulation showing visual turbidity in daylight corresponds to visual score 3). A formulation is classified physical unstable with respect to protein aggregation, when it shows visual turbidity in daylight. Alternatively, the turbidity of the formulation can be evaluated by simple turbidity measurements well-known to the skilled person. Physical stability of the aqueous protein formulations can also be evaluated by using a spectroscopic agent or probe of the conformational status of the protein. The probe is preferably a small molecule that preferentially binds to a non-native conformer of the protein. One example of a small molecular spectroscopic probe of protein structure is Thioflavin T. Thioflavin T is a fluorescent dye that has been widely used for the detection of amyloid fibrils. In the presence of fibrils, and perhaps other protein configurations as well, Thioflavin T gives rise to a new excitation maximum at about 450 nm and enhanced emission at about 482 nm when bound to a fibril protein form. Unbound Thioflavin T is essentially non-fluorescent at the wavelengths.

Other small molecules can be used as probes of the changes in protein structure from native to non-native states. For instance the "hydrophobic patch" probes that bind preferentially to exposed hydrophobic patches of a protein. The hydrophobic patches are generally buried within the tertiary structure of a protein in its native state, but become exposed as a protein begins to unfold or denature. Examples of these small molecular, spectroscopic probes are aromatic, hydrophobic dyes, such as antrhacene, acridine, phenanthroline or the like. Other spectroscopic probes are metal-amino acid complexes, such as cobalt metal complexes of hydrophobic amino acids, such as phenylalanine, leucine, isoleucine, methionine, and valine, or the like.

The term "chemical stability" of the protein formulation as used herein refers to chemical covalent changes in the protein structure leading to formation of chemical degradation products with potential less biological potency and/or potential increased immunogenic properties compared to the native protein structure. Various chemical degradation products can be formed depending on the type and nature of the native protein and the environment to which the protein is exposed. Elimination of chemical degradation can most probably not be completely avoided and increasing amounts of chemical degradation products is often seen during storage and use of the protein formulation as well-known by the person skilled in the art. Most proteins are prone to deamidation, a process in which the side chain amide group in glutaminyl or asparaginyl residues is hydrolysed to form a free carboxylic acid. Other degradations pathways involves formation of high molecular weight transformation products where two or more protein molecules are covalently bound to each other through transamidation and/or disulfide interactions leading to formation of covalently bound dimer, oligomer and polymer degradation products (Stability of Protein Pharmaceuticals, Ahern. T.J. & Manning M.C., Plenum Press, New York 1992). Oxidation (of for instance methionine residues) can be mentioned as another variant of chemical degradation. The chemical stability of the protein formulation can be evaluated by measuring the amount of the chemical degradation products at various time-points after exposure to different environmental conditions (the formation of degradation products can often be accelerated by for instance increasing temperature). The amount of each individual degradation product is often determined by separation of the degradation products depending on molecule size and/or charge using various chromatography techniques (e.g. SEC-HPLC and/or RP-HPLC).

In one aspect of the invention the pharmaceutical formulation comprising the derivative of human amylin or an analogue thereof is stable for more than 6 weeks of usage and for more than 3 years of storage.

In another aspect of the invention the pharmaceutical formulation comprising the derivative of human amylin or an analogue thereof is stable for more than 4 weeks of usage and for more than 3 years of storage.

In a further aspect of the invention the pharmaceutical formulation comprising the derivative of human amylin or an analogue thereof is stable for more than 4 weeks of usage and for more than two years of storage.

In an even further aspect of the invention the pharmaceutical formulation comprising the derivative of human amylin or an analogue thereof is stable for more than 2 weeks of usage and for more than two years of storage.

In another aspect, the present invention relates to a derivative according to the invention for use as a medicament.

In one aspect, a derivative according to the invention is used for the preparation of a medicament for the treatment or prevention of hyperglycemia, type 2 diabetes, impaired glucose tolerance, type 1 diabetes, obesity, hypertension, syndrome X, dyslipidemia, cognitive disorders, atherosclerosis, myocardial infarction, stroke, coronary heart disease and other cardiovascular disorders, inflammatory bowel syndrome, dyspepsia and gastric ulcers.

In another aspect, a derivative according to the invention is used as a medicament for delaying or preventing disease progression in type 2 diabetes.

In another aspect, a derivative according to the invention is used as a medicament for decreasing food intake, decreasing β-cell apoptosis, increasing β-cell function and β-cell mass, and/or for restoring glucose sensitivity to β-cells.

In one aspect of the invention, the derivative according to the invention is for use as a medicament for the treatment or prevention of hyperglycemia, type 2 diabetes, impaired glucose tolerance, type 1 diabetes, obesity, hypertension, syndrome X, dyslipidemia, cognitive disorders, atherosclerosis, myocardial infarction, coronary heart disease and other cardiovascular disorders, stroke, inflammatory bowel syndrome, dyspepsia and gastric ulcers or for delaying or preventing disease progression in type 2 diabetes or for decreasing food intake, decreasing β-cell apoptosis, increasing β-cell function and β-cell mass, and/or for restoring glucose sensitivity to β-cells, is provided.

In a further aspect of the invention, a method for the treatment or prevention of hyperglycemia, type 2 diabetes, impaired glucose tolerance, type 1 diabetes, obesity, hypertension, syndrome X, dyslipidemia, cognitive disorders, atherosclerosis, myocardial infarction, coronary heart disease and other cardiovascular disorders, stroke, inflammatory bowel syndrome, dyspepsia and gastric ulcers or for delaying or preventing disease progression in type 2 diabetes or for decreasing food intake, decreasing β-cell apoptosis, increasing β-cell function and β-cell mass, and/or for restoring glucose sensitivity to β-cells, the method comprising administering to a patient in need of such treatment an effective amount for such treatment of a derivative according to the invention, is provided.

The treatment with a derivative according to the present invention may also be combined with a second or more pharmacologically active substances, e.g. selected from antidiabetic agents, antiobesity agents, appetite regulating agents, antihypertensive agents, agents for the treatment and/or prevention of complications resulting from or associated with diabetes and agents for the treatment and/or prevention of complications and disorders resulting from or associated with obesity. Examples of these pharmacologically active substances are: Insulin, sulphonylureas, biguanides, meglitinides, glucosidase inhibitors, glucagon antagonists, DPP-IV (dipeptidyl peptidase-IV) inhibitors, inhibitors of hepatic enzymes involved in stimulation of gluconeogenesis and/or glycogenolysis, glucose uptake modulators, compounds modifying the lipid metabolism such as antihyperlipidemic agents as HMG CoA inhibitors (statins), compounds lowering food intake, RXR agonists and agents acting on the ATP-dependent potassium channel of the β-cells; Cholestyramine, colestipol, clofibrate, gemfibrozil, lovastatin, pravastatin, simvastatin, probucol, dextrothyroxine, neteglinide, repaglinide; β-blockers such as alprenolol, atenolol, timolol, pindolol, propranolol and metoprolol, ACE (angiotensin converting enzyme) inhibitors such as benazepril, captopril, enalapril, fosinopril, lisinopril, alatriopril, quinapril and ramipril, calcium channel blockers such as nifedipine, felodipine, nicardipine, isradipine, nimodipine, diltiazem and verapamil, and α-blockers such as doxazosin, urapidil, prazosin and terazosin; CART (cocaine amphetamine regulated transcript) agonists, NPY (neuropeptide Y) antagonists, MC4 (melanocortin 4) agonists, orexin antagonists, TNF (tumor necrosis factor) agonists, CRF (corticotropin releasing factor) agonists, CRF BP (corticotropin releasing factor binding protein) antagonists, urocortin agonists, β3 agonists, MSH (melanocyte-stimulating hormone) agonists, MCH (melanocyte-concentrating hormone) antagonists, CCK (cholecystokinin) agonists, serotonin re-uptake inhibitors, serotonin and noradrenaline re-uptake inhibitors, mixed serotonin and noradrenergic compounds, 5HT (serotonin) agonists, bombesin agonists, galanin antagonists, growth hormone, growth hormone releasing compounds, TRH (thyreotropin releasing hormone) agonists, UCP 2 or 3 (uncoupling protein 2 or 3) modulators, leptin agonists, DA agonists (bromocriptin, doprexin), lipase/amylase inhibitors, RXR (retinoid X receptor) modulators, TRβ agonists; histamine H3 antagonists, gastrin and gastrin analogs.

It should be understood that any suitable combination of the derivatives according to the invention with one or more of the above-mentioned compounds and optionally one or more further pharmacologically active substances are considered to be within the scope of the present invention.

*All references, including publications, patent applications, and patents, cited herein are hereby incorporated by reference in their entirety and to the same extent as if each reference were individually and specifically indicated to be incorporated by reference and were set forth in its entirety herein (to the maximum extent permitted by law).*

*All headings and sub-headings are used herein for convenience only and should not be construed as limiting the invention in any way.*

*The use of any and all examples, or exemplary language (e.g., "such as') provided herein, is intended merely to better illuminate the invention and does not pose a limitation on the scope of the invention unless otherwise claimed. No language in the specification should be construed as indicating any non-claimed element as essential to the practice of the invention.*

*The citation and incorporation of patent documents herein is done for convenience only and does not reflect any view of the validity, patentability, and*/*or enforceability of such patent documents.*

*This invention includes all modifications and equivalents of the subject matter recited in the claims appended hereto as permitted by applicable law.*

### ASSAYS

### Pharmacological Assay (I) - Experimental protocol for efficacy testing on appetite with an amylin derivative , using an ad libitum fed rat model.

TAC:SPRD @mol rats or Wistar rats from M&B Breeding and Research Centre A/S, Denmark are used for the experiments. The rats have a body weight 200-250 g at the start of experiment. The rats arrive at least 10-14 days before start of experiment with a body weight of 180-200 g. Each dose of derivative is tested in a group of 6-8 rats. A vehicle group of 6-8 rats is included in each set of testing.

When the animals arrive they are housed individually in a reversed light/dark phase (lights off 7:30 am, lights on 7:30 pm), meaning that lights are off during daytime and on during nighttime. Since rats normally initiate food intake when light is removed, and eat the major part of their daily food intake during the night, this set up results in an alteration of the initiation time for food intake to 7:30 am, when lights are switched off. During the acclimatization period of 10-14 days, the rats have free access to food and water. During this period the animals are handled at least 3 times. The experiment is conducted in the rats' home cages. Immediately before dosing the rats are randomised to the various treatment groups (n=6-8) by body weight. They are dosed according to body weight at between 7:00 am and 7:45 am, with a 0.01-3 mg/kg solution administered intraperitoneally (ip), orally (po) or subcutaneously (sc). The time of dosing is recorded for each group. After dosing, the rats are returned to their home cages, where they then have access to food and water. The food consumption is recorded individually continuously by on-line registration or manually every hour for 7 hours, and then after 24 h and sometimes 48 h. At the end of the experimental session, the animals are euthanised.

The individual data are recorded in Microsoft excel sheets. Outliers are excluded after applying the Grubbs statistical evaluation test for outliers, and the result is presented graphically using the GraphPad Prism program.

### Pharmacological Assay (II) - Experimental protocol for efficacy testing on appetite with an amylin derivative, using a schedule fed rat model.

TAC:SPRD @mol rats or Wistar rats from M&B Breeding and Research Centre A/S, Denmark are used for the experiments. The rats have a body weight 200-250 g at the start of experiment. The rats arrive at least 10-14 days before start of experiment with a body weight of 180-200 g. Each dose of derivative is tested in a group of 6-8 rats. A vehicle group of 6-8 rats is included in each set of testing.

When the animals arrive they are housed individually. At least 7 days prior to onset of study the will start training to a feeding schedule allowing them to have free access to food and water in a scheduled time period between 3 and 7 h. In the remaining period of the day, the rats will not have access to food, but only water. Within a week rats will eat the complete daily ration in the set schedule. Since rats normally initiate food intake when light is removed, and eat the major part of their daily food intake during the night, this set up results allow for monitoring of food intake during day time and will typically mean less variation in the vehicle group compared to an ad libitum fed rat. During the acclimatization period of 10-14 days, the rats have free access to food and water. During this period the animals are handled at least 3 times. The experiment is conducted in the rats' home cages. Immediately before dosing the rats are randomised to the various treatment groups (n=6-8) by body weight. They are dosed according to body weight at between 15 to 30 min prior to given access to food with a 0.01-3 mg/kg solution administered intraperitoneally (ip), orally (po) or subcutaneously (sc). The time of dosing is recorded for each group. After dosing, the rats are returned to their home cages, where they then have access to food and water. The food consumption is recorded individually continuously by on-line registration or manually every hour during the schedule. At the end of the experimental session, the animals are euthanised.

The individual data are recorded in Microsoft excel sheets. Outliers are excluded after applying the Grubbs statistical evaluation test for outliers, and the result is presented graphically using the GraphPad Prism program.

### Amylin receptor binding assay

For the receptor binding assay, membranes from the Amylin 3(a)/CRE-luc cells described below were used. The tracer was Tyr-pramlintide iodinated with ¹²⁵I in the N-terminal tyrosine. SPA-WGA beads (GE Healthcare RPNQ0001) were incubated in a 96 well Optiplate in a buffer containing 50 mM Hepes, 5 mM MgCl₂, 5 mM EGTA, 0.025% Tween-20, pH 7.4 with membranes, tracer and a dilution series of the amylin analog.

After incubation for 2 hours at room temperature the plates were centrifuged and counted on a Topcounter. The EC50 was calculated as a measure of receptor affinity.

### Amylin luciferase assay

### 1. Amylin assay outline

It has previously been published (Poyner DR et al 2002, Pharmacological Reviews 54(2) 233-246) that activation of Amylin receptors (coexpression of Calcitonin receptor and receptor activity modifying peptides RAMPs) by Amylin leads to an increase in the intracellular concentration of cAMP. Consequently, transcription is activated at promotors containing multiple copies of the cAMP response element (CRE). It is thus possible to measure Amylin activity by use of a CRE luciferase reporter gene introduced into BHK cells also expressing an Amylin receptor.

### 2. Construction of an Amylin 3(a)/CRE-luc cell line

A BHK570 cell line stably transfected with the human calcitonin receptor (CTa) and a CRE-responsive luciferase reportergene. The cell line was further transfected with RAMP-3, using standard methods. This turns the Calcitonin receptor into an Amylin 3(a) receptor. Methotrexate, Neomycin, and Hygromycin are selection markers for luciferase, the Calcitonin receptor, and RAMP-3, respectively.

### 3. Amylin luciferase assay

To perform activity assays, BHK Amylin 3(a)//CRE-luc cells were seeded in white 96 well culture plates at a density of about 20.000 cells/well. The cells were in 100 µl growth medium (DMEM with 10% FBS, 1 % Pen/Strep, 1 mM Na-pyruvate, 250 nM Methotrexate, 500 µg/ml Neomycin, and 400 µg/ml Hygromycin). After incubation overnight at 37°C and 5% CO₂, the growth medium was replaced by 50 µl/well assay medium (DMEM (without phenol red), Glumamax^{™}, 10% FBS, and 10 mM Hepes, pH 7,4). Further, 50 µl/well of standard or sample in assay buffer were added. After 4 hours incubation at 37°C and 5% CO₂, the assay medium with standard or sample were removed and replaced by 100 µl/well PBS. Further, 100 µl/well LucLite^{™} was added. The plates were sealed and incubated at room temperature for 30 minutes. Finally, luminescence was measured on a TopCounter (Packard) in SPC (single photon counting) mode.

### EXAMPLES

### Peptide Synthesis:

One method of peptide synthesis was by Fmoc chemistry on a microwave-based Liberty peptide synthesizer (CEM Corp., North Carolina). The resin was Tentagel S RAM with a loading of 0.25 mmol/g. The coupling chemistry was DIC/HOAt in NMP using amino acid solutions of 0.3 M in NMP and a molar excess of 6-8 fold. Coupling conditions was 5 minutes at up to 70°C. Deprotection was with 5% piperidine in NMP at up to 70°C. When a chemical modification of a lysine side chain was desired, the lysine was incorporated as Lys(mtt) and the N-terminal amino acid was protected by treatment with Boc-carbonate. The mtt group was repomed by suspending the resin in neat hexafluoroisopropanol for 20 minutes followed by washing with DCM and NMP. The chemical modification of the lysine was performed either by manual synthesis or by one or more automated steps on the Liberty followed by a manual coupling. Another method of peptide synthesis was by Fmoc chemistry on an ABI 433 with HBTU coupling. After synthesis the resin was washed with DCM and dried, and the peptide was cleaved from the resin by a 2 hour treatment with TFA/TIS/water (92.5/5/2.5) followed by precipitation with diethylether. After further washing with diethylether and drying, the peptide was redissolved in water at 1-2 mg/ml, pH adjusted to about 4.5, and the disulfide bridge formed by treatment with 1 eq. of [Pt(IV)ethylenediamine₂Cl₂]Cl₂ overnight. The peptide was immediately purified by standard RP-HPLC on a C18 column using acetonitrile/TFA. Alternatively, the disulfide bridge was formed on the resin by treatment with 10 equivalents of iodine in NMP for 1 hour and in this case the peptide was purified directly after cleavage and ether precipitation. The identity of the peptide was confirmed by MALDI-MS.

### Abbreviations used:

DMF: N,N dimethylformamide
HBTU: 2-(1H-Benzotriazol-1-yl-)-1,1,3,3 tetramethyluronium hexafluorophosphate
Fmoc: 9 H-fluoren-9-ylmethoxycarbonyl
Boc: tert butyloxycarbonyl
Mtt: 4-methyltrityl
DCM: dichloromethane
TIS: triisopropylsilane)
TFA: trifluoroacetic acid
NMP: 1-Methyl-pyrrolidin-2-one
HOAt: 1-Hydroxy-7-azabenzotriazole
DIC: Diisopropylcarbodiimide

### EXAMPLES 1-29

The derivatives of example 1-29 as described in below table 1 were prepared as described above under "Peptide Synthesis". The derivatives were tested in the "Amylin luciferase assay" as described above and the results are shown in table 1. The receptor affinities of the derivatives were tested in the "Amylin receptor binding assay" as described above and the results are shown in table 1. The HPLC elution data in table 1 were measured in an analytical HPLC-system using an acetonitrile/TFA gradient from 10% to 90% over 20 minutes.

All peptide sequences 1-16 have a disulfide bridge between the cysteines (positions 2 and 7) and all are C-terminal amides.

**Table 1**

| Example No. | Seq # (see below table 2) | R-group (see below table 3) | Attachment point (alpha means the N-terminus) | HPLC elution (% acetonitrile) | Potency luciferase assay | Receptor affinity |
|---|---|---|---|---|---|---|
| 1 | 16 | B | alpha | 65 | * | * |
| 2 | 2 | B | 3 | 60 | ** | *** |
| 3 | 2 | A | 3 | 61 | ** | *** |
| 4 | 3 | C | 29 | 66 | * | * |
| 5 | 3 | B | 29 | 64 | * | * |
| 6 | 3 | G | 29 | 65 | * | *** |
| 7 | 4 | G | 21 | 66 | ** | *** |
| 8 | 5 | G | 17 | 63 | ** | *** |
| 9 | 7 | A | 25 | 62 | ** | *** |
| 10 | 6 | G | 25 | 64 | ** | *** |
| 11 | 2 | H | 3 | 58 | * | * |
| 12 | 2 | I | 3 | 61 | * | * |
| 13 | 2 | G | 3 | 62 | ** | *** |
| 14 | 2 | C | 3 | 62 | ** | *** |
| 16 | 8 | D | alpha | 63 | *** | *** |
| 17 | 8 | E | alpha | 61 | * | * |
| 18 | 9 | D | 3 | 61 | * | * |
| 19 | 9 | H | 3 | 61 | * | * |
| 20 | 9 | E | 3 | 59 | * | * |
| 21 | 10 | B | alpha | 61 | *** | *** |
| 22 | 11 | B | 3 | 60 | ** | *** |
| 23 | 12 | D | 3 | 60 | ** | |
| 24 | 8 | B | alpha | 63 | ** | *** |
| 25 | 13 | C | alpha | 64 | * | ** |
| 26 | 6 | F | 25 | 59 | * | * |
| 27 | 6 | D | 25 | 62 | ** | *** |
| 28 | 14 | D | 3 | 61 | ** | *** |
| 29 | 15 | B | 3 | 63 | * | * |

**Table 2**

| Seq # | Peptide sequence | SEQ ID No. |
|---|---|---|
| 01 | KCNTATCATQRLANFLVHSSNNFGPILPPTNVGSNTY | pramlintide |
| 02 | KCKTATCATQRLANFLVHSSNNFGPILPPTNVGSNTY | K3 |
| 03 | KCNTATCATQRLANFLVHSSNNFGPILPKTNVGSNTY | K29 |
| 04 | KCNTATCATQRLANFLVHSSKNFGPILPPTNVGSNTY | K21 |
| 05 | KCNTATCATQRLANFLKHSSNNFGPILPPTNVGSNTY | K17 |
| 06 | KCNTATCATQRLANFLVHSSNNFGKILPPTNVGSNTY | K25 |
| 07 | KCNTATCATQRLANFLVRSSNNFGKILPPTNVGSNTY | R18,K25 |
| 08 | CNTATCATQRLANFLVRSSNNLGPVLPPTNVGSNTY | desK1,R18,L23,V26 |
| 09 | KCKTATCATQRLANFLVPSSNNFGPILPPTNVGSNTY | K3,P18 |
| 10 | KCNTATCATQRLANFLVRSSNNFGPILPPTNVGSNTY | R18 |
| 11 | KCKTATCATQRLANFLVRSSNNFGPILPPTNVGSNTY | K3,R18 |
| 12 | KCGTATCATQRLANFLARSSNNFGPILSPTNVGSNTY | G3,A17,R18,S28 |
| 13 | EERECNTATCATQRLANFLVHSSNNFGPILPPTNVGSNTY | EER-E1 |
| 14 | KCKTATCATQRLANFLVRSSQNLGPVLPPTNVGSNTY | K3,R18,Q21,L23,V26 |
| 15 | ECKTATCATQRLANFLVHSSNNFGPILPPTNVGSNTY | E1,K3 |
| 16 | EECNTATCATQRLANFLVHSSNNFGPILPPTNVGSNTY | E-E1 |

**Table 3**

| R-group | |
|---|---|
| A | 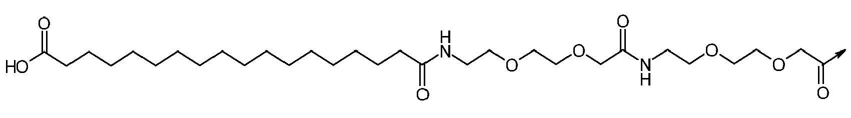 |
| B | 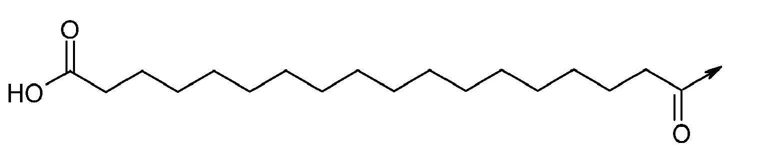 |
| C | 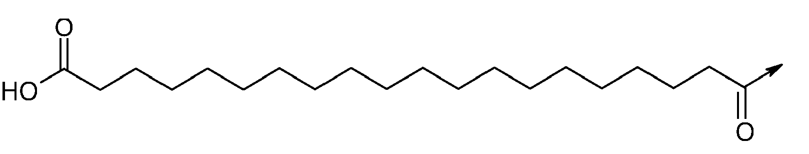 |
| D | 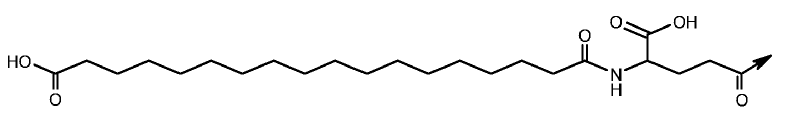 |
| E | 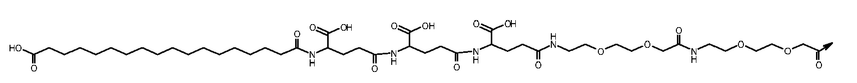 |
| F | 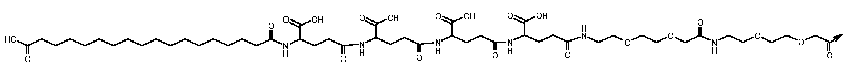 |
| G | 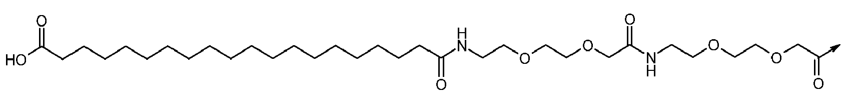 |
| H | 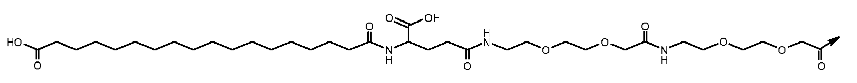 |
| I | 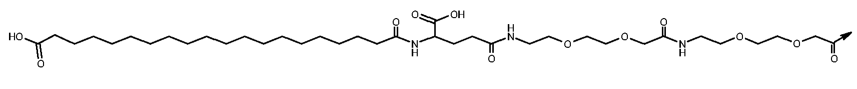 |

In the above table 1 the potency of the derivatives was measured in the "Amylin luciferase assay" as described above under "Assays". In the above table 1 the affinity of the derivatives was measured in the "Amylin receptor binding assay" as described above under "Assays".

In the above table 1 "***" indicates a potency/affinity comparable to pramlintide, "**" indicates a potency/affinity somewhat lower than pramlintide, and "*" indicates a potency/affinity much lower than pramlintide.

## Claims

1. A derivative of human amylin with SEQ ID NO:17 or an analogue thereof, wherein
a) the amino acid residue in position 1 is any natural amino acid which is connected to a N-terminal extension consisting of 1-10 amino acids, wherein
a. said extension is further linked to an albumin binding residue or a polyethylene glycol polymer, or
b. an amino acid residue in position 2-37 has been substituted with a lysine residue, and said lysine residue is linked to an albumin binding residue or a polyethylene glycol polymer, optionally via a linker;
or
b) the amino acid residue in position 3, 21, 25 or 29 has been substituted with a lysine residue and wherein said lysine residue is linked to an albumin binding residue or a polyethylene glycol polymer, optionally via a linker;
or
c) the amino acid residue in position 18 is arginine, and an amino acid residue in position 1-17 or 19-37 has been substituted with a lysine residue, and wherein said lysine residue is linked to an albumin binding residue or a polyethylene glycol polymer, optionally via a linker;
wherein the amino acid numbering conforms with the amino acid numbering in SEQ ID NO:17.

2. A derivative of human amylin with SEQ ID NO:17 or an analogue thereof according to claim 1, wherein
the amino acid residue in position 1 is any natural amino acid which is connected to a N-terminal extension consisting of 1-10 amino acids, wherein
a. said extension is further linked to an albumin binding residue or a polyethylene glycol polymer, or
b. an amino acid residue in position 2-37 has been substituted with a lysine residue, and said lysine residue is linked to an albumin binding residue or a polyethylene glycol polymer, optionally via a linker;
and optionally the amino acid residue in position 18 is arginine;
wherein the amino acid numbering conforms with the amino acid numbering in SEQ ID NO:17.

3. A derivative of human amylin with SEQ ID NO:17 or an analogue thereof according to claim 1, wherein
the amino acid residue in position 3, 21, 25 or 29 has been substituted with a lysine residue and wherein said lysine residue is linked to an albumin binding residue or a polyethylene glycol polymer, optionally via a linker;
and optionally the amino acid residue in position 18 is arginine;
wherein the amino acid numbering conforms with the amino acid numbering in SEQ ID NO:17.

4. The derivative according to any one of claims 1-3, wherein the derivative has from 1-12 amino acid substitutions compared to human amylin.

5. The derivative according to any one of claims 1-4, wherein the derivative is an analogue of human amylin of SEQ ID NO:17, wherein 0-8 additional charges have been added compared to human amylin.

6. A derivative according to claim 1 comprising an amino acid sequence of formula 1: 
Xaa1-Cys- Xaa3-Thr-Ala-Thr-Cys-Ala-Thr-Gln-Arg-Leu-Ala-Asn-Phe-Leu- Xaa17-Xaa18-
Ser-Ser- Xaa21-Asn- Xaa23-Gly- Xaa25- Xaa26-Leu-Xaa28-Xaa29-Thr-Asn-Val-Gly-Ser-Asn-
Thr-Tyr-Xaa38
Formula (1) (SEQ ID No:18)
 wherein
Xaa₁ is deleted or independently selected from Lys and Glu;
Xaa₃ is independently selected from Asn, Gly and Lys;
Xaa₁₇ is independently selected from Ala, Val and Lys;
Xaa₁₈ is independently selected from His, Pro and Arg;
Xaa₂₃ is independently selected from Phe and Leu;
Xaa₂₁ is independently selected from Asn, Gln and Lys;
Xaa₂₅ is independently selected from Ala, Pro and Lys;
Xaa₂₆ is independently selected from Val and Ile;
Xaa₂₈ is indenpendently selected from Ser and Pro;
Xaa₂₉ is independently selected from Ser, Pro and Lys;
the C-terminal may optionally be derivatized as an amide;
the N-terminal may optionally be extended with 1-10 amino acid residues wherein if Xaa₁ is Lys and Xaa₃ is Asn and Xaa₂₁ is Asn and Xaa₂₅ is Ala or Pro, then Xaa₂₉ is Lys; and
if Xaa₁ is Glu, then said Glu is connected to a N-terminal extension consisting of 1-10 amino acids, and said extension is further linked to an albumin binding residue or a polyethylene glycol polymer; and
if Xaa₁ is Lys, then one amino acid in a position selected from the group consisting of Xaa₁, Xaa₃, Xaa₂₁, Xaa₂₅ and Xaa₂₉ in formula (1) is linked to an albumin binding residue or a polyethylene glycol polymer, optionally via a linker.

7. A pharmaceutical composition comprising a derivative according to any one of the claims 1-6, and a pharmaceutically acceptable excipient.

8. A derivative according to any one of the claims 1-6 for use as a medicament.

9. A method for the treatment, prevention or alleviation of hyperglycemia, type 2 diabetes, impaired glucose tolerance, type 1 diabetes, obesity, syndrome X or dyslipidemia in a subject comprising administering to a subject a derivative according to any one of claims 1-6 or a pharmaceutical composition according to claim 7.

10. A process for preparing a pharmaceutical composition according to claim 7 comprising mixing a derivative according to any one of claims 1-6 with pharmaceutically acceptable substances and/or excipients.

11. Method for increasing the time of action in a patient of human amylin or an analog thereof, **characterized by** modifying
a) the amino acid residue in position 1 by optionally substituting with any natural amino acid, and by connecting said natural amino acid to a N-terminal extension consisting of 1-10 amino acids, and further
a. linking said extension to an albumin binding residue or a polyethylene glycol polymer, or
b. modifying an amino acid residue in position 2-37 by substitution with a lysine residue, and by linking said lysine residue to an albumin binding residue or a polyethylene glycol polymer, optionally via a linker,
or;
b) the amino acid residue in position 3, 21, 25 or 29 by substitution with a lysine residue and by linking said lysine residue to an albumin binding residue or a polyethylene glycol polymer, optionally via a linker,
or;
c) the amino acid residue in position 18 by substitution with an arginine residue and an amino acid residue in position 2-17 or 19-37 by substitution with a lysine residue and by linking said lysine residue to an albumin binding residue or a polyethylene glycol polymer, optionally via a linker;
wherein the amino acid numbering conforms with the amino acid numbering in SEQ ID NO:17.


SEQUENCE LISTING

<110>  Novo Nordisk A/S

<120>  IMPROVED DERIVATIVES OF AMYLIN

<130>  7710.000-EP

<160>  18

<170>  PatentIn version 3.4

<210>  1
<211>  37
<212>  PRT
<213>  Artificial

<220>
<223>  Amylin derivative sequence

<400>  1

Lys Cys Asn Thr Ala Thr Cys Ala Thr Gln Arg Leu Ala Asn Phe Leu
1               5                   10                  15

Val His Ser Ser Asn Asn Phe Gly Pro Ile Leu Pro Pro Thr Asn Val
            20                  25                  30

Gly Ser Asn Thr Tyr
        35

<210>  2
<211>  37
<212>  PRT
<213>  Artificial

<220>
<223>  Amylin derivative sequence

<400>  2

Lys Cys Lys Thr Ala Thr Cys Ala Thr Gln Arg Leu Ala Asn Phe Leu
1               5                   10                  15

Val His Ser Ser Asn Asn Phe Gly Pro Ile Leu Pro Pro Thr Asn Val
            20                  25                  30

Gly Ser Asn Thr Tyr
        35

<210>  3

<211>  37
<212>  PRT
<213>  Artificial

<220>
<223>  Amylin derivative sequence

<400>  3

Lys Cys Asn Thr Ala Thr Cys Ala Thr Gln Arg Leu Ala Asn Phe Leu
1               5                   10                  15

Val His Ser Ser Asn Asn Phe Gly Pro Ile Leu Pro Lys Thr Asn Val
            20                  25                  30

Gly Ser Asn Thr Tyr
        35

<210>  4
<211>  37
<212>  PRT
<213>  Artificial

<220>
<223>  Amylin derivative sequence

<400>  4

Lys Cys Asn Thr Ala Thr Cys Ala Thr Gln Arg Leu Ala Asn Phe Leu
1               5                   10                  15

Val His Ser Ser Lys Asn Phe Gly Pro Ile Leu Pro Pro Thr Asn Val
            20                  25                  30

Gly Ser Asn Thr Tyr
        35

<210>  5
<211>  37
<212>  PRT
<213>  Artificial

<220>
<223>  Amylin derivative sequence

<400>  5

Lys Cys Asn Thr Ala Thr Cys Ala Thr Gln Arg Leu Ala Asn Phe Leu
1               5                   10                  15

Lys His Ser Ser Asn Asn Phe Gly Pro Ile Leu Pro Pro Thr Asn Val
            20                  25                  30

Gly Ser Asn Thr Tyr
        35

<210>  6
<211>  37
<212>  PRT
<213>  Artificial

<220>
<223>  Amylin derivative sequence

<400>  6

Lys Cys Asn Thr Ala Thr Cys Ala Thr Gln Arg Leu Ala Asn Phe Leu
1               5                   10                  15

Val His Ser Ser Asn Asn Phe Gly Lys Ile Leu Pro Pro Thr Asn Val
            20                  25                  30

Gly Ser Asn Thr Tyr
        35

<210>  7
<211>  37
<212>  PRT
<213>  Artificial

<220>
<223>  Amylin derivative sequence

<400>  7

Lys Cys Asn Thr Ala Thr Cys Ala Thr Gln Arg Leu Ala Asn Phe Leu
1               5                   10                  15

Val Arg Ser Ser Asn Asn Phe Gly Lys Ile Leu Pro Pro Thr Asn Val
            20                  25                  30

Gly Ser Asn Thr Tyr
        35

<210>  8
<211>  36
<212>  PRT
<213>  Artificial

<220>
<223>  Amylin derivative sequence

<400>  8

Cys Asn Thr Ala Thr Cys Ala Thr Gln Arg Leu Ala Asn Phe Leu Val
1               5                   10                  15

Arg Ser Ser Asn Asn Leu Gly Pro Val Leu Pro Pro Thr Asn Val Gly
            20                  25                  30

Ser Asn Thr Tyr
        35

<210>  9
<211>  37
<212>  PRT
<213>  Artificial

<220>
<223>  Amylin derivative sequence

<400>  9

Lys Cys Lys Thr Ala Thr Cys Ala Thr Gln Arg Leu Ala Asn Phe Leu
1               5                   10                  15

Val Pro Ser Ser Asn Asn Phe Gly Pro Ile Leu Pro Pro Thr Asn Val
            20                  25                  30

Gly Ser Asn Thr Tyr
        35

<210>  10
<211>  37
<212>  PRT
<213>  Artificial

<220>
<223>  Amylin derivative sequence

<400>  10

Lys Cys Asn Thr Ala Thr Cys Ala Thr Gln Arg Leu Ala Asn Phe Leu
1               5                   10                  15

Val Arg Ser Ser Asn Asn Phe Gly Pro Ile Leu Pro Pro Thr Asn Val
            20                  25                  30

Gly Ser Asn Thr Tyr
        35

<210>  11
<211>  37
<212>  PRT
<213>  Artificial

<220>
<223>  Amylin derivative sequence

<400>  11

Lys Cys Lys Thr Ala Thr Cys Ala Thr Gln Arg Leu Ala Asn Phe Leu
1               5                   10                  15

Val Arg Ser Ser Asn Asn Phe Gly Pro Ile Leu Pro Pro Thr Asn Val
            20                  25                  30

Gly Ser Asn Thr Tyr
        35

<210>  12
<211>  37
<212>  PRT
<213>  Artificial

<220>
<223>  Amylin derivative sequence

<400>  12

Lys Cys Gly Thr Ala Thr Cys Ala Thr Gln Arg Leu Ala Asn Phe Leu
1               5                   10                  15

Ala Arg Ser Ser Asn Asn Phe Gly Pro Ile Leu Ser Pro Thr Asn Val
            20                  25                  30

Gly Ser Asn Thr Tyr
        35

<210>  13
<211>  40
<212>  PRT
<213>  Artificial

<220>
<223>  Amylin derivative sequence

<400>  13

Glu Glu Arg Glu Cys Asn Thr Ala Thr Cys Ala Thr Gln Arg Leu Ala
1               5                   10                  15

Asn Phe Leu Val His Ser Ser Asn Asn Phe Gly Pro Ile Leu Pro Pro
            20                  25                  30

Thr Asn Val Gly Ser Asn Thr Tyr
        35                  40

<210>  14
<211>  37
<212>  PRT
<213>  Artificial

<220>
<223>  Amylin derivative sequence

<400>  14

Lys Cys Lys Thr Ala Thr Cys Ala Thr Gln Arg Leu Ala Asn Phe Leu
1               5                   10                  15

Val Arg Ser Ser Gln Asn Leu Gly Pro Val Leu Pro Pro Thr Asn Val
            20                  25                  30

Gly Ser Asn Thr Tyr
        35

<210>  15
<211>  37
<212>  PRT
<213>  Artificial

<220>
<223>  Amylin derivative sequence

<400>  15

Glu Cys Lys Thr Ala Thr Cys Ala Thr Gln Arg Leu Ala Asn Phe Leu
1               5                   10                  15

Val His Ser Ser Asn Asn Phe Gly Pro Ile Leu Pro Pro Thr Asn Val
            20                  25                  30

Gly Ser Asn Thr Tyr
        35

<210>  16
<211>  38
<212>  PRT
<213>  Artificial

<220>
<223>  Amylin derivative sequence

<400>  16

Glu Glu Cys Asn Thr Ala Thr Cys Ala Thr Gln Arg Leu Ala Asn Phe
1               5                   10                  15

Leu Val His Ser Ser Asn Asn Phe Gly Pro Ile Leu Pro Pro Thr Asn
            20                  25                  30

Val Gly Ser Asn Thr Tyr
        35

<210>  17
<211>  37
<212>  PRT
<213>  Homo Sapiens

<400>  17

<210>  18
<211>  37
<212>  PRT
<213>  Artificial

<220>
<223>  Amylin derivative sequence

<220>
<221>  VARIANT
<222>  (1)..(1)
<223>  deleted or Lys or Glu

<220>
<221>  VARIANT

<222>  (3)..(3)
<223>  Asn, Gly or Lys

<220>
<221>  VARIANT
<222>  (17)..(17)
<223>  Ala, Val or Lys

<220>
<221>  VARIANT
<222>  (18)..(18)
<223>  His, Pro or Arg

<220>
<221>  VARIANT
<222>  (21)..(21)
<223>  Asn, Gln or Lys

<220>
<221>  VARIANT
<222>  (23)..(23)
<223>  Phe or Leu

<220>
<221>  VARIANT
<222>  (25)..(25)
<223>  Ala, Pro or Lys

<220>
<221>  VARIANT
<222>  (26)..(26)
<223>  Val or Ile

<220>
<221>  VARIANT
<222>  (28)..(28)
<223>  Ser or Pro

<220>
<221>  VARIANT
<222>  (29)..(29)
<223>  Ser, Pro or Lys

<400>  18

Xaa Cys Xaa Thr Ala Thr Cys Ala Thr Gln Arg Leu Ala Asn Phe Leu
1               5                   10                  15

Xaa Xaa Ser Ser Xaa Asn Xaa Gly Xaa Xaa Leu Xaa Xaa Thr Asn Val
            20                  25                  30

Gly Ser Asn Thr Tyr
        35